# EUROPEAN PATENT SPECIFICATION

(11) **EP 1 170 581 B1**
(45) Date of publication and mention of the grant of the patent: **05.05.2010**
(21) Application number: 01112844.4
(22) Date of filing: 31.05.2001
(51) Int. Cl.: G01N 3/40, G01N 33/02

(54) **Apparatus and method for the automatic measurement of the ripening degree of vegetable products**
Vorrichtung und Verfahren zur automatischen Messung des Reifungsgrades von Gemüseprodukten
Appareil et procédé de mesure automatique du degré de maturation de produits végétaux

(30) Priority: 03.07.2000 IT PN000040
(43) Date of publication of application: 09.01.2002
(73) Proprietor: Unitec S.r.l., 48100 Ravenna (IT)
(72) Inventor: Benedetti, Angelo, 48010 Savarna (IT)
(74) Representative: Giugni, Valter

(56) References cited:
- WO-A-98/40737
- WO-A-98/52037
- FR-A- 2 595 824
- FR-A- 2 704 461
- US-A- 4 061 020
- PATENT ABSTRACTS OF JAPAN vol. 014, no. 040 (P-0995), 25 January 1990 (1990-01-25) & JP 01 274059 A (MAKI SEISAKUSHO:KK), 1 November 1989 (1989-11-01)

## Description

The present invention refers to an improved apparatus for the automatic measurement of the ripening degree of individual vegetable products, in which this apparatus may be included in a comprehensive plant for conveying and selectively sorting said individual vegetable products in accordance with both said ripening degree and other properties, e.g. the weight, thereof.

The main purpose of the apparatus according to the present invention is to be able to perform non-destructive tests on said vegetable products on a continuous basis, i.e. on a continuous sequence of said products as they are moved along by a moving conveyor, in which each such product is arranged in an individual accommodating cell in said conveyor and is tested one at a time. The apparatus according to the present invention is also adapted to supply real-time data to appropriate control and actuation members in view of further possible selection and sorting operations in accordance with the outcome of each single measurement so carried out on each single vegetable product.

A number of types of apparatuses and related methods are known in the art, which are capable of measuring the ripening degree of a vegetable product, in particular a fruit, by measuring the property that most of all is indicative of such a ripening degree, and namely the relative hardness of the outer surface of the fruit under a definite pressure being applied on to a delimited zone of said surface of the fruit.

So, for instance, the solution disclosed in the patent publication WO 98 52037 illustrates, to say it in just a few words, a device for measuring the ripening degree of a vegetable product, in which such a measurement is carried out by the application of a light pressure between a contact element and a delimited zone of the surface of the vegetable product.

Such a contact element is connected to a piezoelectric cell, so that, from the comparison of the resulting forces, as combined with other variables, the hardness of the product and, therefore, the ripening degree thereof is inferred.

This patent is characterized at least in that:
- the sensing element penetrates the surface of the fruit, and therefore it at least partly, and the data that is detected and processed is a force, as measured by means of a piezoelectric cell. Furthermore, the positioning of the sensor is carried out by first putting under pressure and then evacuating, i.e. creating a vacuum condition inside a sealed elastic bellows-like element.

This solution turns therefore out as not only being practically far from being perfect, but also scarcely acceptable in mass measurement applications involving very large volumes of fruit pieces to be measured. It furthermore requires a considerable complexity of its construction and an equally considerable extent of fine tuning as far as its overall adjustment is concerned.

The US patent 4,061,020 to The Regents of the University of California discloses a particularly interesting solution, in which some sensors are brought into simple contact, without exerting any pressure, with the fruit whose ripening degree is to be measured on the basis of the extent of yielding of the surface thereof. Upon having so established a contact, without any pressure, with the surface of the fruit, and therefore having so established a "zero" reference position, a sensor is pushed with a pre-defined, known force into the product, so as to penetrate it down to a certain depth: from the difference between such two positions of the sensor, and in accordance with the applied force, the hardness of the surface of the fruit is calculated and, as a result, the ripening degree thereof is assessed and rated.

The solution according to this patent is quite obviously more respectful of the integrity of the fruit, since a known and pre-defined pressure is actually exerted on to the surface thereof. However, especially in the case of very ripe, mellow and, therefore, particularly yielding fruit pieces it may occur that such a pressure turns actually out to be excessive and, as a consequence, fully capable of anyway damaging, i.e. spoiling the fruit.

Furthermore, the use of at least two sensors and the operation that must be performed to subtract the respective yielding extents considerably add to the complexity of such an apparatus that is scarcely suitable for mass-measurement applications.

The invention described in WO 94/29715 consists in imparting a mechanical stress to the fruit being measured, in order to detect its elastic reaction thereto by means of other sensors associated to piezoelectric transducers that are sensitive to the force exerted on them by the fruit, as well as to the intensity and the characteristics of such a force.

Said piezoelectric transducer delivers a corresponding signal, the analysis of which then delivers the required information on the quality of the fruit.

It should be noticed that even this apparatus, and related method, according to the disclosure in the above cited patent stands clearly out for its marked complexity. Furthermore, the elastic reaction that has ultimately to be measured might even fail to occur in some cases, i.e. does not assuredly occur in all cases, or it might even prove such as to scarcely or inadequately correlate with the actual ripening degree of the fruit.

The US patent 5,315,879, assigned to the "Centre National du Machinisme Agricole du Génie Rural des Eaux et des Forêts" in France, discloses a cylindrically shaped, pressure actuated sensor which is in the first place and mainly intended to measure the size of the vegetable being measured, and in the second place to measure the degree of hardness and, therefore, ripening thereof.

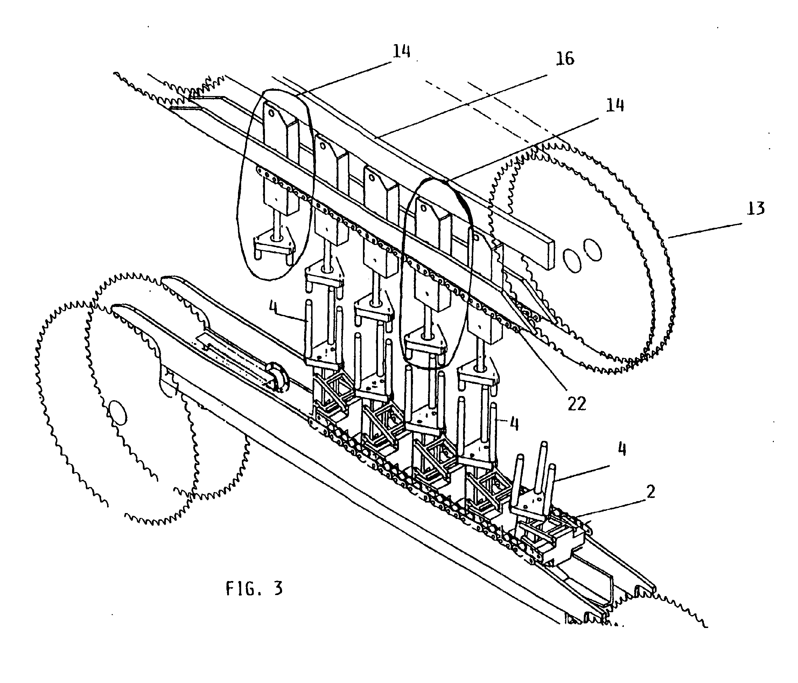

However, although such a patent claims that the measurement of the hardness is a non-destructive one, as this emerges from the illustration in the corresponding Figure 3 there occurs actually a penetration of a sensor in the fruit and this unavoidably leads to some, albeit slight extent of detriment of the integrity of the fruit.

Known from the disclosure in EP 0 351 430 and US 4,542,639 is the method consisting in measuring the ripening degree of a fruit (or the degree of tenderness or softness of a structure) through the creation of an impact against the surface of the item being measured by means of a special structure, of the percussion hammer type, while detecting and recording the elastic reaction waves, By mathematically processing (using the Fourier transform) the respective characteristics, a frequency spectrum is obtained which is then compared with a "sample frequency spectrum".

Also the above cited patents, although quite sophisticated from a technical point of view, suffer from the same drawbacks as the preceding ones, and namely a great deal of complexity and a not really soft manner in handling the fruit.

The patent EP 0 439 405, filed in the name of Institut National de la Recherche Agronomique and the Centre Technique Interprofessionel des fruits et Legumes, appears on the contrary to be quite interesting, since it refers to the measurement of the characteristics of vegetable products by means of a penetrometer associated to electronic processing and appropriate software means.

However, the solution disclosed in such a patent is based also on the use of "... a worm screw ..." and, therefore, on a kind of technical solution that may be advantageously used in laboratory testing applications, but surely not to mass measurement purposes involving large volumes of fruit products moving on a conveyor belt or similar handling means.

From WO 98/40737 it is divulged a kind of method and apparatus for assessing the ripeness of a fruit or vegetable, consisting of an impactor able and controlled in order to strike the fruit, and provided with a transducer which produces an electrical output signal representing the reaction force generated by said impact, wherein said signal is in the form of an electrical pulse representing said reaction force generated by the item under test; however two main drawbacks can be experienced by said method:
- the first drawback is due to the fact that, in order to be actually and faithfully representative of the fruit ripeness, said impact, generated by a properly activated arm, shall strike the fruit with a not negligible force; therefore the fruits, particularly the ripe fruits, may be unacceptably damaged, particularly when the same fruits are offered as "first choice fruits";
- the second drawback depends on the circumstance that the kind of test method, i.e. the processing of a signal representing the reaction force to the impact, shows intrinsically uncertain due to the circumstance that the time length of the impact, and therefore of the measured signal, is extremely short, and so not representative of the fruit reaction in regard of a progressive but softer pressure.-

From FR 2 595 824 it is divulged a kind of method able to measure the ripeness of a fruit; however this method is only a lab method, fully unsuited for processing the fruits in a conveying and production line.-

From JP 01274059 it is divulged an apparatus and method for detecting the condition of vegetables and fruits moving in a conveyor; however the internal quality inspecting device inspects the shock sound when the vegetable is slightly hit, and analyzes the sound and outputs a grade signal; said method is apparently fully inadequate to be used in a production plant, where the environmental noise is generally at high level and absolutely not predictable.-

From WO 98/52037 it is divulged an assembly and method for assessing the condition of a vegetable comprising plunger means adapted to bring a passive sensor into contact with that vegetable to produce a signal related to a specific property of that vegetable; however the sensor is only able to detect the type of a specific characteristic, as may be a gas, an Infra red emission, a change in a capacitance, an optical property or a force reaction to an impactor showed by that vegetable.-

However said characteristic do not represent what it is actually wanted to measure, i.e. the general ripeness provided by the measurement of the penetration degree of a sensor into the fruit on a not negligible time length and under soft pressure.-

Moreover said patent, as far as the impact force is concerned, is apparently identical to the above cited WO 98/40737.-

From US 4, 061, 020 it is known a kind of deformeter for testing the maturity of fruit, based on exerting a predetermined pressure on it; however the measure is taken only on a specific position of the fruits which are moving along a conveyer, and therefore the time length of the measure, and as an obvious consequence its precision, is quite low.-

From FR 2 704 461 it is known an apparatus an method for measuring the sizes and the weights on a sequence of vegetables along a moving conveyor; however no provision is given in order to measure their ripeness.-

It is therefore desirable, and it is actually a main purpose of the present invention, to provide a technical solution for application to apparatuses for the automatic measurement of the ripening degree of a continuous sequence of vegetable products, which is absolutely simple and low-cost, but at the same time accurate and effective in minimizing any damage which the products may possibly be exposed to. Furthermore, such a solution shall be capable of operating with the means conveying the individual products moving at differentiated speeds.

Such an aim of the present invention, along with further features thereof, is reached in an apparatus that is made and operates as recited in the appended claims.

Anyway, features and advantages of the present invention may be more readily understood from the description that is given below by way of non-limiting example with reference to the accompanying drawings, in which:
- Figure 1 is a schematical view, as seen from a side thereof, of a part of the apparatus according to the present invention;
- Figure 2 is a partially lateral perspective view of the apparatus illustrated in Figure 1;
- Figure 3 is a perspective view of the same apparatus, however as seen form the opposite side with respect to the illustration in Figure 2;
- Figure 4 is a cross-sectional view of the apparatus according to the section F - F of Figure 1;
- Figures 5 and 6 are views of two successive state or trim conditions of the apparatus illustrated in Figure 4, during the operation thereof;
- Figure 7 is a perspective view of a portion of the apparatus illustrated in Figure 3;
- Figure 8 is a view of an improved variant of the device illustrated in Figure 7;
- Figures 9 and 10 are schematical outer front views of a device of the apparatus according to two different, but complementary operating modalities;
- Figures 9a and 10a are cross-sectional views of the respective devices illustrated in Figures 9 and 10;

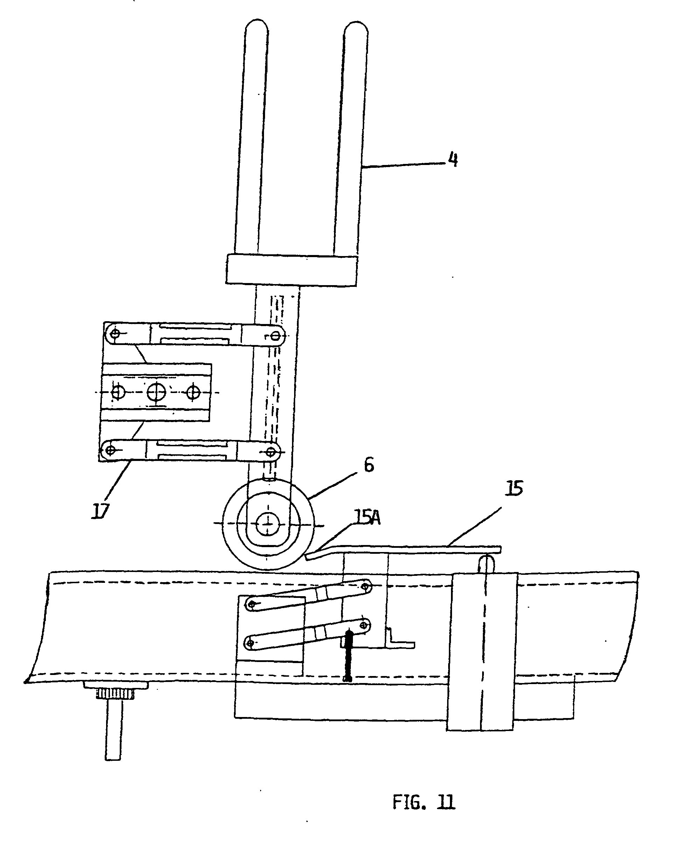

- Figure 11 is a side view of a detail of the apparatus according to the present invention;
- Figures 12 and 13 are two side views of some basic parts of an apparatus according to the present invention; and
- Figure 14 is a view of a reciprocally arranged variant of some parts of an apparatus according to the present invention.

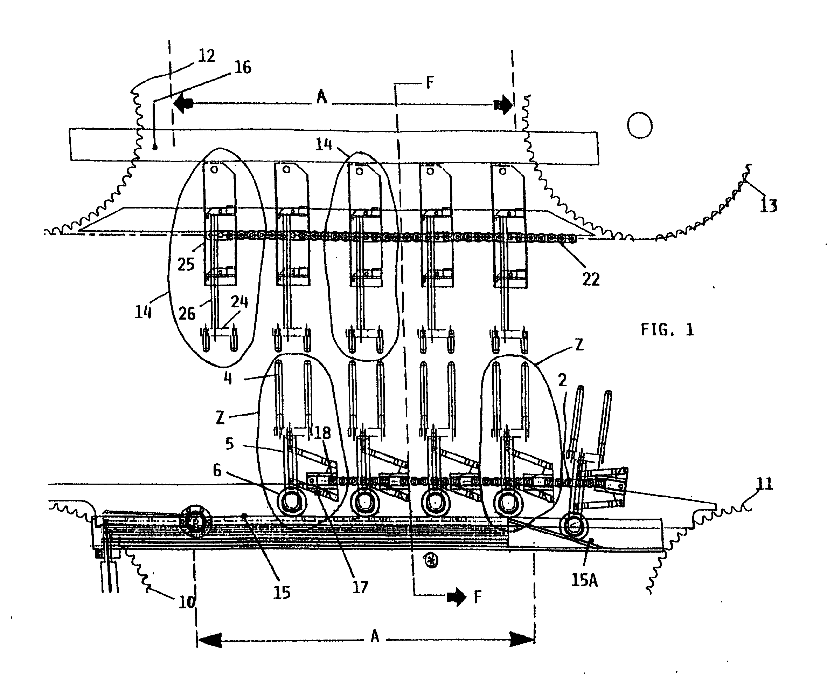

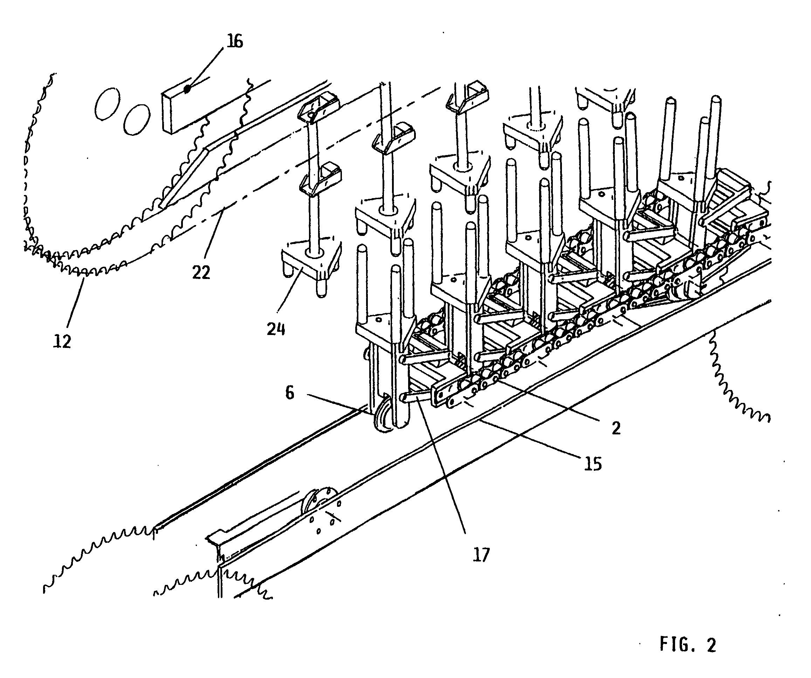

With reference to the above listed Figures 1, 2 and 3, it can be noticed that an apparatus according to an exemplified embodiment of the present invention comprises:
- a first moving chain 2 arranged along the path covered by the individual products 3 conveyed thereon, and driven by two gearwheels, i.e. a driving and a driven one, 10 and 11, respectively, which cause the chain to move along an upper, substantially horizontal stretch A, in which the measurement on the surface of the product is actually carried out, as well as a return stretch extending below said upper stretch A and not shown in the Figures;
- a plurality of assemblies Z carrying said individual products, in which each such assembly is represented by the zone encircled by the closed-loop line generally indicated at Z in Figure 1; these assemblies are substantially constituted by a cradle-like member 4, which will be described in greater detail further on and is adapted to support a respective individual product 3, a rod 5 sustaining said cradle-like member, at the lower end portion thereof there is provided a wheel 6 resting and sliding on a track 15 (see description further on), as well as a parallelogram-shaped structure 17 connecting such a rod 5, and via a pin 18, to respective engagement positions of said first moving chain 2;
- a second moving chain 22 that is again arranged along the path covered by the individual products 3 conveyed thereon, but in a position lying above said individual products 3, and is in turn driven by two respective gearwheels, i.e. a driving and a driven one 12, 13, respectively;
- a plurality of assemblies 14 connected to said second moving chain 22, the basic features of which will be described in greater detail further on;
- an elongated guiding member 16 lying above said assemblies 14, in which the lower surface 29 of said guiding member 16 is a flexible surface with controlled and pre-definable resistance, wherein such a resistance is capable of being set through the use of various means. In a preferred manner, such a controlled resistance is capable of being set, and therefore obtained, by arranging inside said elongated guiding member 16 a sealed, pressure-tight tubular member 30 that is adapted to be inflated to pre-definable pressure values in such a manner as to cause said tubular member 30 to come partially into contact with said lower surface 29, to which it then transfers the elastic resistance due to its internal pressure;
- a resting and sliding track 15, on which the afore cited wheels 6 are able to rest and slide; such a track also comprises an access ramp 15A adapted to enable the wheels 6 to move up to the substantially plane and horizontal portion of the track 15, which corresponds to both the length and the alignment of said stretch A of the chain and, of course on an upper level, to said elongated member 16.

Furthermore, said gearwheels and the support structures of the just above described members are joined to a framework or load-bearing structure (not shown).

Said assemblies 14, which comprise a device for detecting their respective position on the vertical plane, as this will be described in greater detail further on, are adapted to come into a sliding or rolling contact with said lower surface 29 and to send an information on their vertical position to a device 33 provided to detect the position on the vertical plane of each one of said assemblies 14, when these assemblies move through a pre-determined fixed position.

In other words, and with particular, although not sole reference to Figures 1, 2 and 3, it can more concisely and plainly be stated that the members indicated below are arranged, from bottom to top, in the following order:
- the track 15 with the associated boarding or access ramp,
- the first chain 2,
- the whole of said assemblies Z which are aligned on top, but rest with their wheel 6 on said track 15,
- said assemblies 14 which are aligned at the bottom,
- said second chain 22,
- said guiding member 16.

In addition there is the assembly formed by a sequence of individual products 3, preferably singularised fruits, arranged and held, from the bottom, by a respective one of said assemblies Z and, from the top, by a respective one of said assemblies 14, in which both said assemblies are driven to move horizontally in parallel along the stretch A with a synchronous motion, so that, within such a stretch, the individual product 3 is caused to move forward with a continuous motion, but is kept in a stable position with respect to the corresponding pair of assemblies Z and 14. Anyway, their exact and mutual arrangement can be clearly observed in the above cited Figures, the illustrations of which are such as to fully remove any possible doubt or uncertainty in this connection.

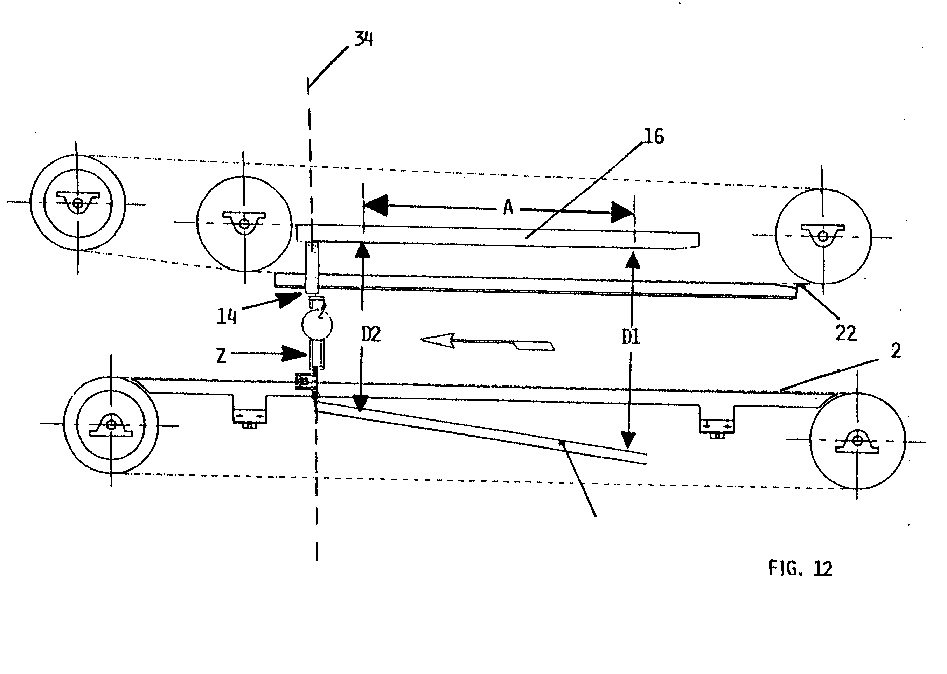

With reference to Figures 12, 13 and 14, the track 15 and the elongated member 16 can be noticed to be arranged in a manner which is not parallel, but slightly convergent, so that, following the direction of movement of said cradles 4, the distance D1, as measured between the track 15 and the guiding element 16 at the beginning of the horizontal path covered by the cradle, in practice between two reference points in the stretch A, is slightly greater than the corresponding distance D2 at the end of the stretch A between the corresponding reference points.

The two moving chains 2 and 22 are caused to rotate at the same speed by the respective gearwheels, and are arranged above each other along two parallel and superposed paths for at least the length of said guiding member 16 and said track 15.

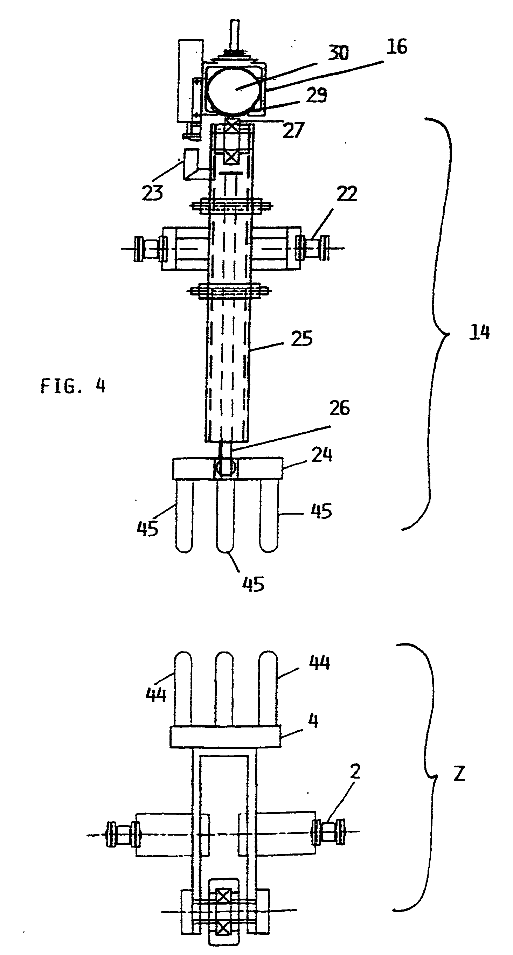

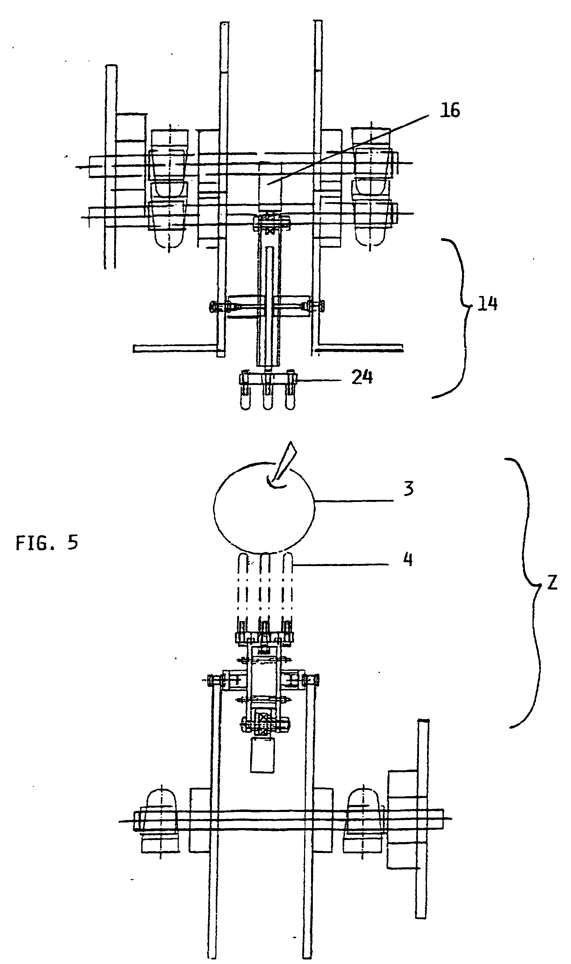

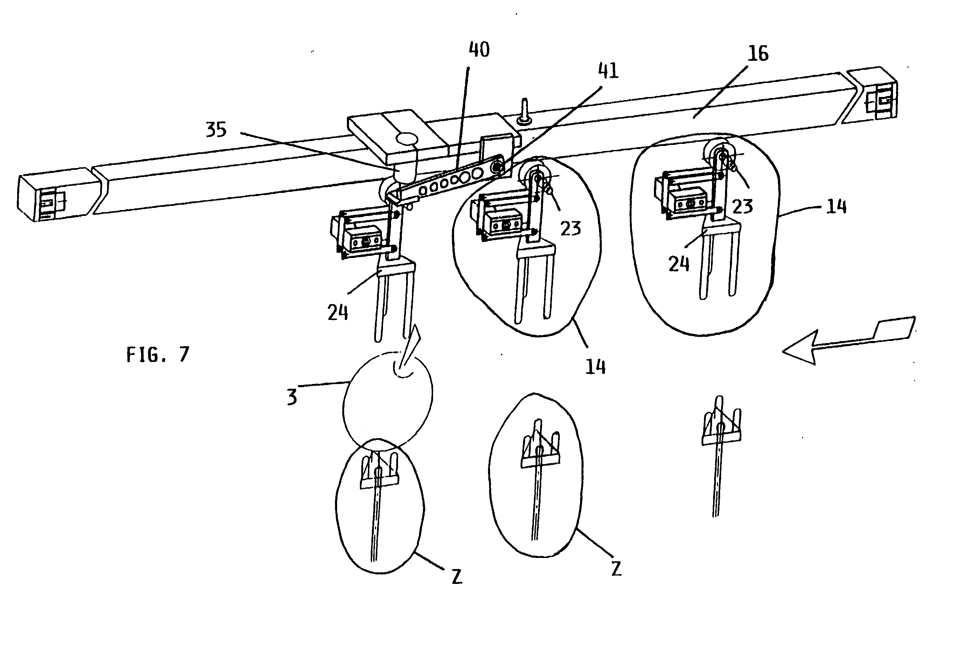

With reference to Figures 1, 6 and 13, the assemblies Z and 14 as defined above are provided in an arrangement formed by superposed pairs, so that each assembly Z is surmounted by a respective assembly 14, with which it is then able to move along synchronically owing to the two chains 2 and 22 moving at the same speed, as this is illustrated in particular in Figures 1, 4, 5 and 7.

Each assembly 14 is comprised of at least a moving means 24, of the feeling probe type, a lock/release box 25, an actuation rod 26 connecting the feeler probe 24 to the respective box 25, a contact member 27 attached, with respect to said box, on the opposite side with respect to the one on which there is provided the respective feeler probe 24; finally, firmly joined to a respective box 25 there is provided a further positioning element 23 which is oriented upwards and is adapted to intercept, at a certain point along the path, an appropriate device 33 and to displace it in such a manner as to enable such a displacement to be detected and measured, as this will be explained in greater detail further on.

Since each one of said assemblies 14 is joined to the related chain 22, and the latter is driven to move along a closed-loop path symmetrically with the first chain 2 with respect to a horizontal plane passing through at approximately the same height as the individual products 3, each such assembly 14 is caused to successively rotate about the gearwheel 13 and, therefore, is successively transferred on to the lower side of said path, so that said assembly 14 places itself in a position lying above the respective assembly Z all along the stretch in which said chains are facing each other; in this stretch, said assembly 14 takes of course a certain trim, which is the one shown in Figures 1, 2 and 3 with the feeler probe 24 below and the box 25 above.

When, owing to the chain 22 being so driven, the assembly is brought on to the upper stretch of the chain, then its position is inverted, thereby placing the feeler probe 24 above and the box 25 below.

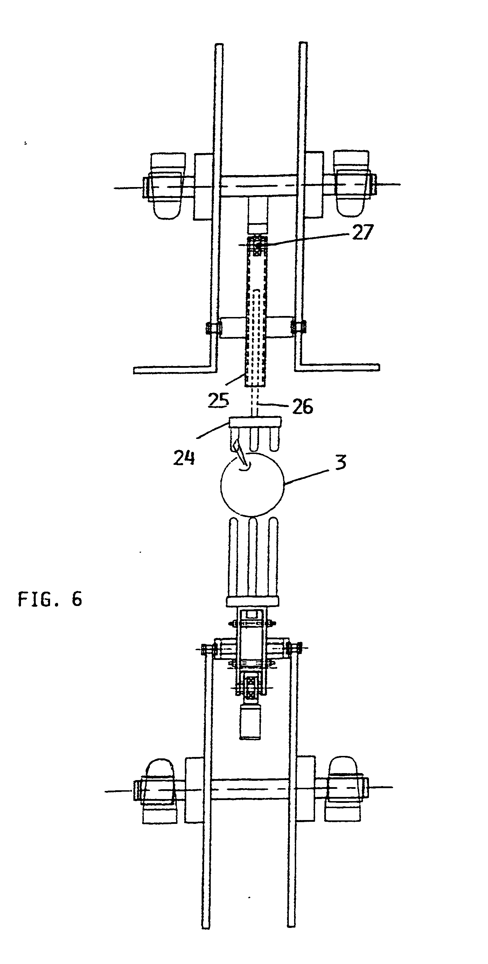

A further basic feature which is common to said assemblies 14 is given by the fact that, owing to gravity, when each one of said assemblies comes to be associated to the upper side of the chain 22, the feeler probe 24 thereof and the related actuation rod 26 substantially retract by gravity into the box 25, whereas, when by rotating about the gearwheel it is then displaced on to the lower side of the chain 22, the feeler probe 24 is capable of falling freely out downwards until it reaches an obstacle that stops it from falling any further, such as this is illustrated in Figure 6.

From this moment on, the box 25 is immediately enabled to automatically take a trim or state in which it locks the respective feeler probe 24 in the full-out lowered position which the latter has in the meanwhile extended to, and which is not changed or altered if the feeler probe is pushed again upwards, while however always keeping said assembly on the lower side of the chain 22 that causes it to move on.

The mechanism that allows said rod 26 to freely slide downwards, while on the other side preventing it from moving up again, is contained in the box 25 and, owing to its being largely known in the art, shall not be described here any further.

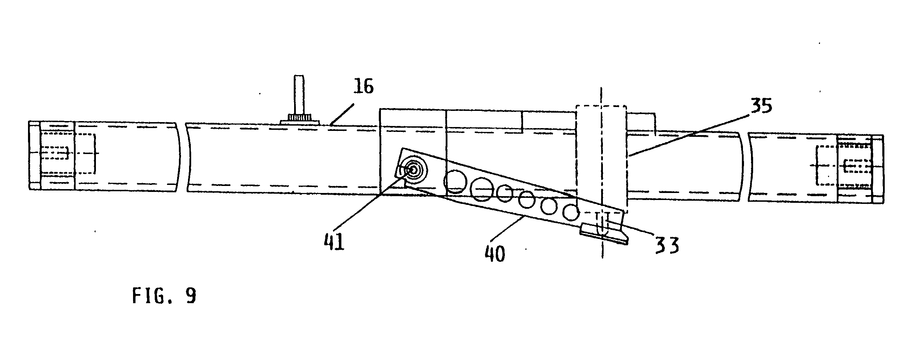

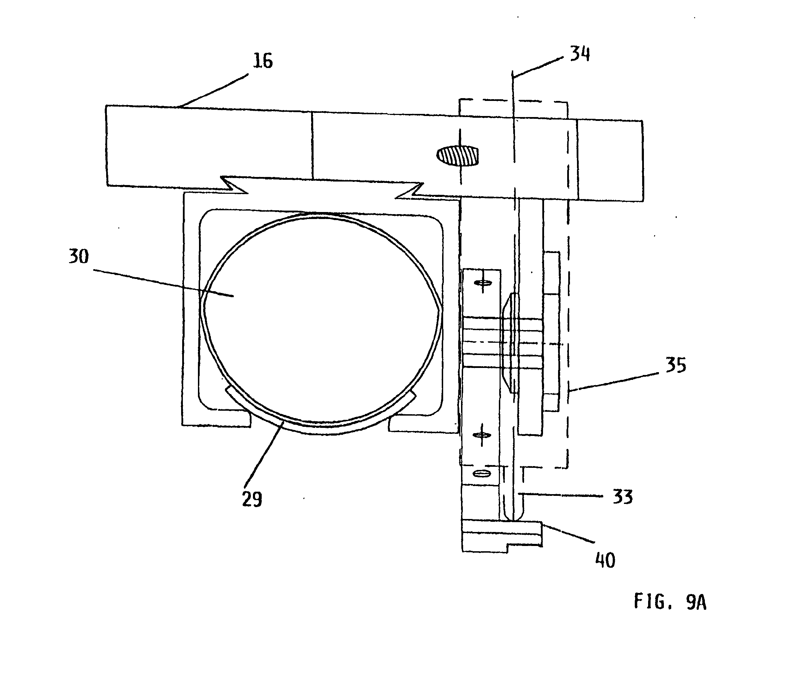

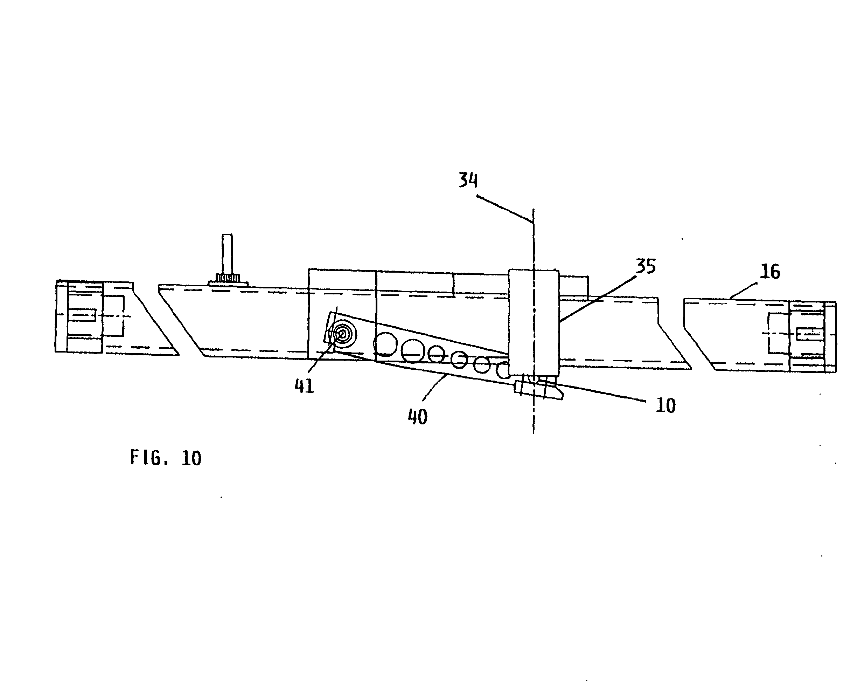

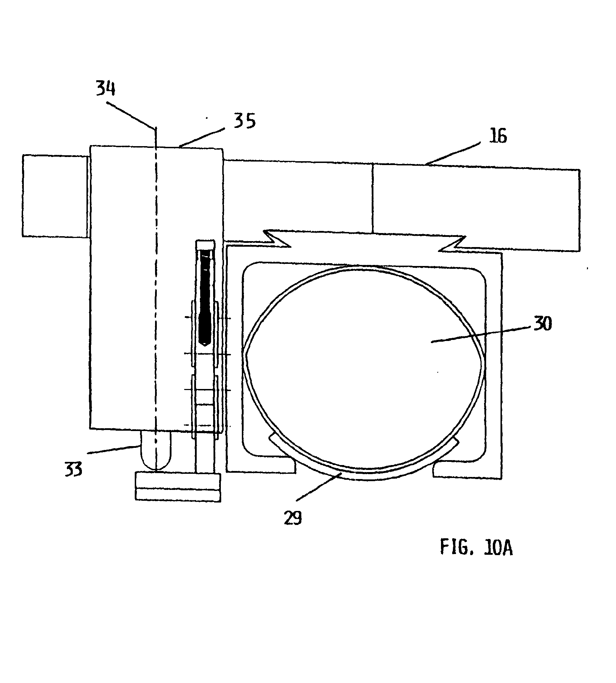

The device 33 which, at the end of the measurement stretch and, therefore, in a pre-determined position 34, is adapted to record the vertical position that is successively taken by said assemblies 14, may be implemented in any of a number of ways: one of these ways, as shown in Figures 9A and 10A, is represented by a pin 33 capable of being inserted in a means 35 adapted to measure the depth of penetration of such a pin 33, and therefore the extent to which the respective assembly 14 raises, and conclusively delivers, along with other parameters, an information that depends on the degree of resistance of the surface of the individual product being tested.

In addition, said guiding member 16 is provided, in said measurement position 34 thereof, with a means 40 which is illustrated in the Figures as a lever hinged on a point 41 located in said guiding member 16.

The purpose of said means 40 is to intercept said positioning element 23 and to transfer its reciprocal motion, which is produced by the mutually interfering positions between said element and said means 40, to said device 33 and, conclusively, to said means 35 for measuring the penetration depth.

At this point of the description, those skilled in the art will certainly be capable to clearly infer how the described apparatus substantially works:
- with the two chains moving in a uniform and continuous manner, with said assemblies Z and 14 arranged on the respective chains and capable of being suitable aligned in pairs on the vertical plane, the individual products 3 are sequentially inserted, using largely known means that will therefore not be described here, in said cradles 4; as these cradles then move on, as driven by the motion of the chain 2, the respective assembly Z is first pushed forward, by passing over the ramp 15A, on to the track 15 where the cradle 4 takes a position exactly on the vertical of the corresponding assembly 14, but above all at a precisely preestablished vertical distance from the lower surface 29 and, therefore, the guiding member 16.

From this position, the assembly releases from the box 25 the respective actuation rod 26 with the related feeler probe 24, which, falling down by gravity, eventually strikes against the upward facing surface of the respective individual product 3, and in this position it then stops without any possibility for it to be pushed again upwards.

In such a position, therefore, the individual product 3 is locked in place by two elements, i.e. a lower one, or the cradle 4, and an upper one, or the feeler probe 24, which elements are mutually defined by a distance that depends on the downward stopping position of the respective feeler probe.

As the two chains 2 and 22 keep moving on, the convergence of the described members 15 and 16 generates a gradually increasing pressure exerted by the cradle 4 and the feeler probe 24 on the individual product 3, in which such a pressure relieves itself both through a limited impression on, or deformation of, the outer surface of the product 3, and a limited, but perceptible raising of the assembly 14 that in this ways moves to press with its contact member 27, in a sliding or rolling manner, against said lower surface 29 contiguous to the tubular member 30. Owing to said pressure, said lower surface 29 undergoes a slight deformation, thereby causing the whole corresponding assembly 14 to rise with respect to the guiding member 16.

At the end of the travel along the stretch A, and owing to said members 15 and 16 converging in the afore described manner, the assembly will have risen by a certain extent with respect to said guiding member 16, so that a simple device adapted to measure the extent of such a raising and transfer the related information, along with other data, such as in particular the one relating to the pressure inside the tubular member 30 that co-determines the extent of such a raising, is actually all that is required to calculate, with such automatic computing means as largely known in the art, the degree of resistance to a controlled pressure of the surface of the individual product 3 and, conclusively, to assess the ripening degree of such a product.

Such a simple device is the device 33, associated to the detection means 35, as well as to said positioning elements 23 associated to respective assemblies 14. When performing their motion, each one of said positioning elements 23 successively intercepts said single means 40 joined to said guiding member 16, so that said device 33 is displaced with respect to the detection and indicating means 35.

Such an information is therefore capable of being used for automatically selecting and sorting the individual fruits or individual products 3 in accordance with the respective measured/calculated ripening degree thereof. In which such a selection and sorting, along with the subsequent differentiated conveyance, may be implemented with the use of means that are largely known in the art, which therefore shall not be described here any further.

Figures 9 and 9A illustrate enlarged views of some of the afore described members and parts when the device 33 is in a resting position, i.e. is not engaged by the positioning element 23, whereas Figures 10 and 10A illustrate the same enlarged views when the devices 33 are retracted into the respective means 35 owing to the pushing action of the respective positioning elements 23.

The described invention allows for a number of advantageous variants and improvements.

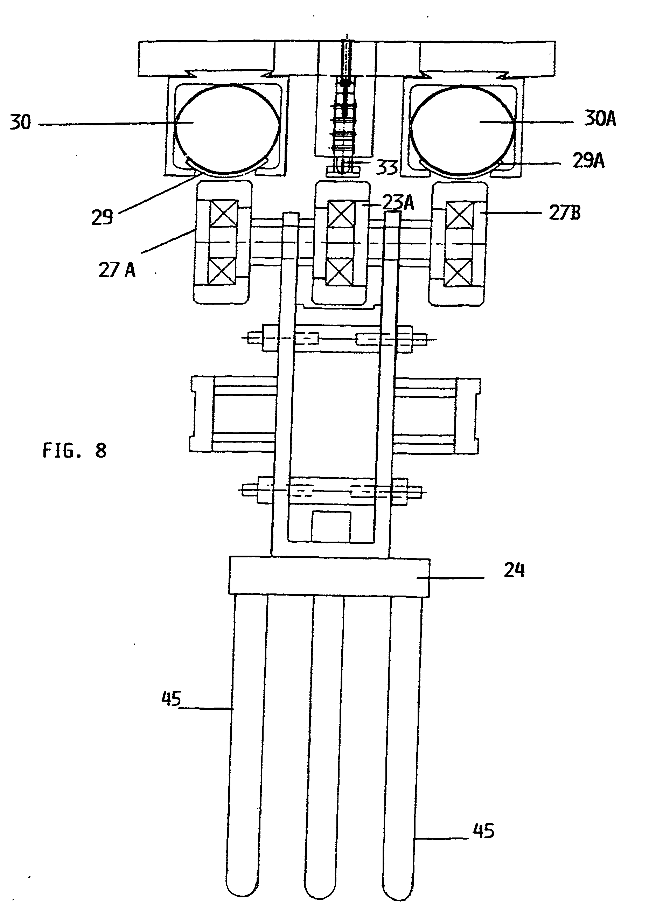

One such improvement consists (see Figure 8) in adopting not just a single tubular member 30, but rather two similar members, in which there are illustrated two parallel pneumatic tubular members 30 and 30A and two respective lower surfaces 29 and 29A, further of course to the support and auxiliary members associated therewith, as illustrated in Figure 8.

It has in fact been noticed that the use of a single tubular member 30 is quite likely to lead to a situation of asymmetry of the force transmitted by the contact members 27, under resulting misalignment of the members comprised in the assembly 14, and conclusively to an incorrect measurement of the displacement on the vertical of the same assembly and, therefore, to a recording error by the device 33.

In view of doing away with such a drawback, the elimination is advantageously proposed of any form of dissymmetry of the assembly 14 with respect to a vertical plane passing substantially through the centre of the individual products 3 during the measurement thereof, while providing said assemblies 14 in a manner that is substantially symmetrical to said plane as defined just above, in particular through the use of two tubular elements 30 and 30A arranged on the opposite sides at the same distance from said plane and, above all, at the same height thereof; furthermore, the means provided to come into contact with the elastic tubular members are in this case constituted by a pair of revolving wheels 27A and 27B. In such a case, even the other above described members associated therewith can be suitably adapted; in particular, the positioning element 23 can be advantageously replaced by a middle wheel 23A to the purpose of reducing frictions. As far as the other members of the construction, it can be readily appreciated, based on the afore given explanations, that the implementation of such an assembly is fully within the ability of those skilled in the art, so that such an issue shall not be dealt with here any further.

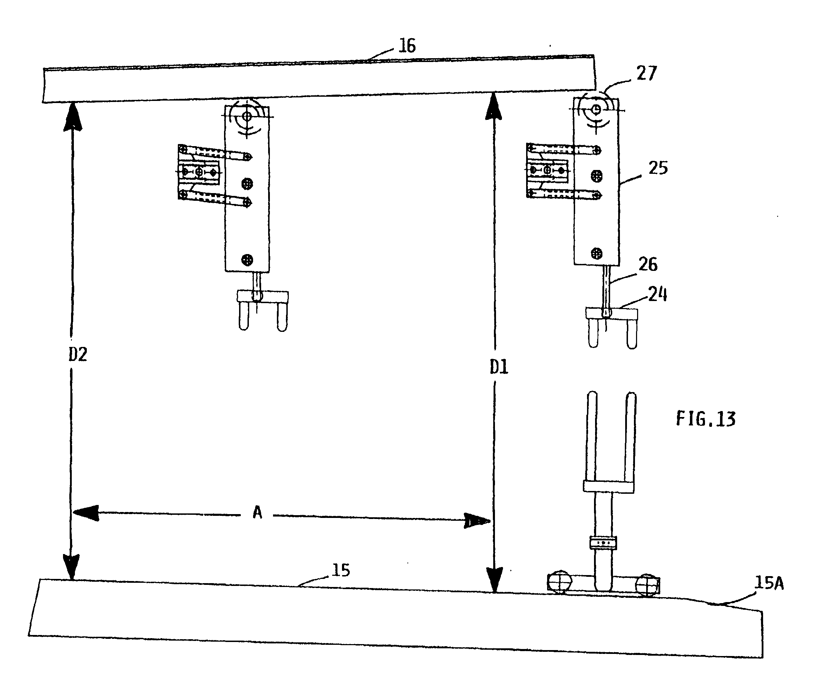

Figures 12 and 13 are overall views of the most important component parts contributing to the implementation of the present invention: clearly visible are, with the chains 2 and 22 and the respective wheels, preferably gear or sprocket wheels, also the guiding member 16 and the resting track 15, which are convergent with respect to each other, as well as an assembly Z and a corresponding assembly 14 which, with a combined action, lock in place and carry a respective individual product 3 along the path along which said individual product 3 is gradually pressed until it reaches the position 34 at which the level of surface deformation thereof is measured.

It should be noticed, in particular in Figure 13, that, although the mutual position of the lower track 15 and the upper guiding member 16 remains unaltered, the parallelograms are now applied between the upper assemblies 14 and the upper chain 22 (not shown).

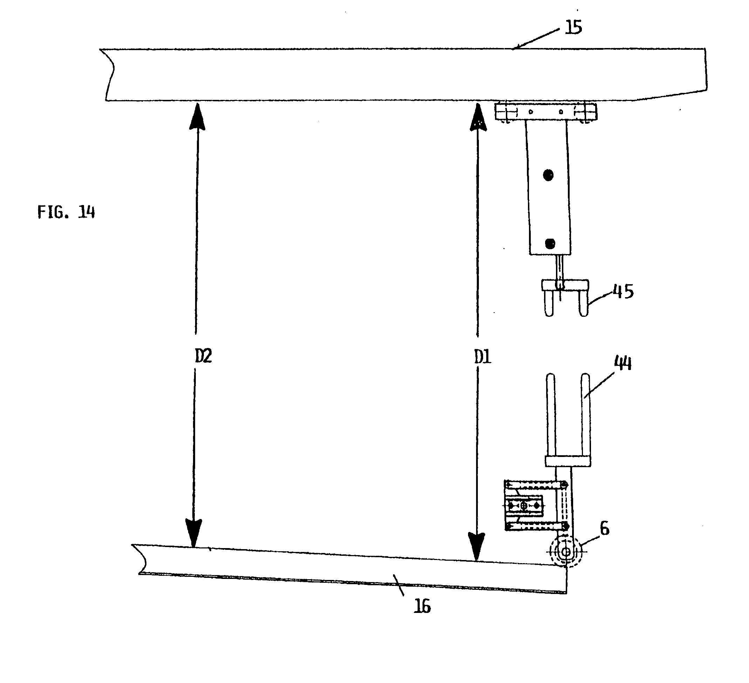

Finally, Figure 14 is a view of a construction variant of the present invention. In this case, in fact, an inversion is made between the position of the track 15 and the guiding member 16, so that also other members that are functionally associated therewith require a corresponding adaptation. It can be readily appreciated that, in this case, on the guiding member 16 weigh both the weight of the fruit and the pressure exerted on the latter by the assembly 14 due to the convergence between said members 15 and 16, so that the elastic portion of said guiding member 16 is forced to yield to an extent that exceeds the yield due to the sole pressure exerted on the individual product 3. Such a drawback could anyway be partly corrected through an adequate processing of the measures being made, and could be advantageously accepted against a simplification in the construction and a reduction in the accuracy requirements as far as the determination of the degree of ripening is concerned.

A further improvement can be obtained by making said cradles with three preferably parallel resting members 44 oriented towards said individual product 3. Such an arrangement allows for combining the best possible backing of the individual product and the least resistance to deformation of the latter, as well as damaging the same product to the smallest possible extent.

The same solution can be advantageously adopted, for the same reasons, for said moving means 24, which are in this case made up by a plurality of preferably parallel rods 45 oriented coaxially with respect to said three resting members.

This technique and the related apparatus, although extensively experimented with altogether favourable results, has anyway turned out as being associated with some drawbacks, which are essentially ascribable to a practical impossibility for the pressure exerted by the feeler probes to be controlled in a strict and most accurate manner, as well as the variability of the hardness of the surface of the fruit depending basically on the zone onto which said pressure is exerted.

In view of doing away with these drawbacks, a solution has been studied and experimented for a long time, which eventually led to the following improvement that will be more readily understood in its features and advantages from the description that is given below by way of a non-limiting example thereof and with reference to the accompanying drawings, in which:

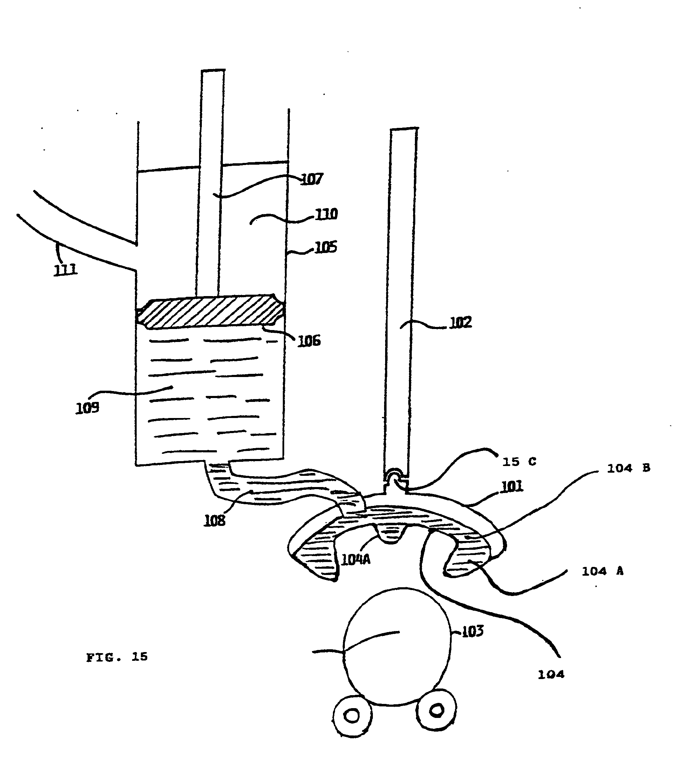

- Figure 15 is a merely symbolical view of a portion of an apparatus according to the present invention, in a first operating state thereof;

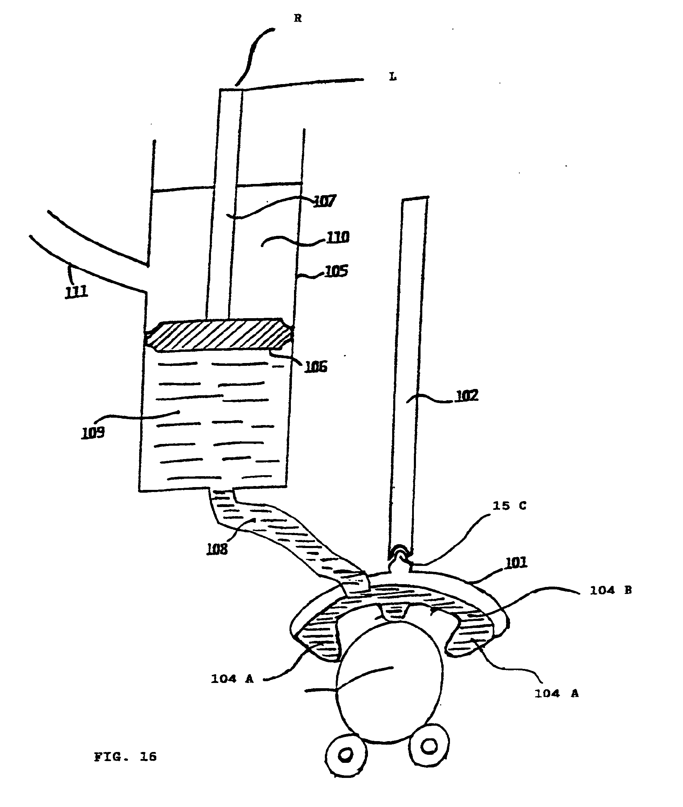

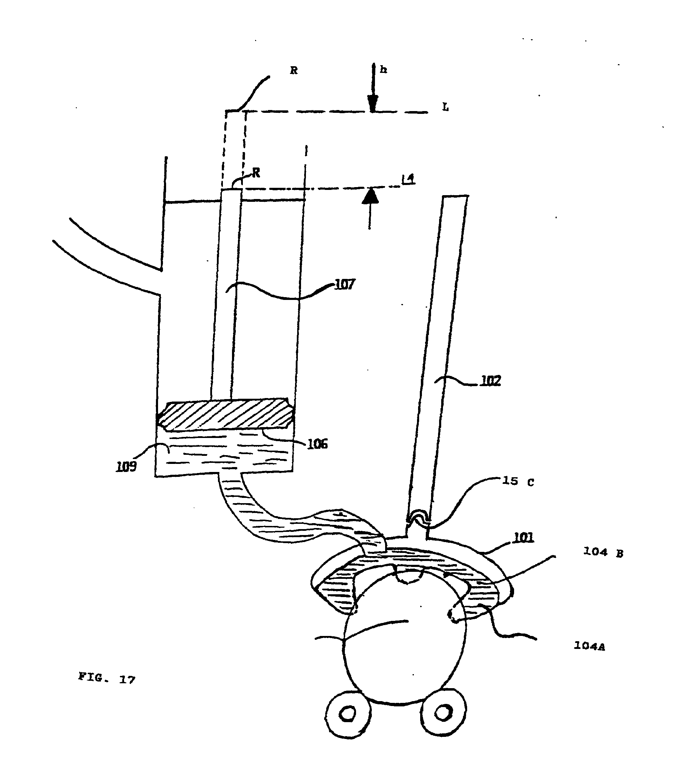

- Figures 16 and 17 are views of the same apparatus as illustrated in Figure 15, but with the apparatus set in respective different operating states;

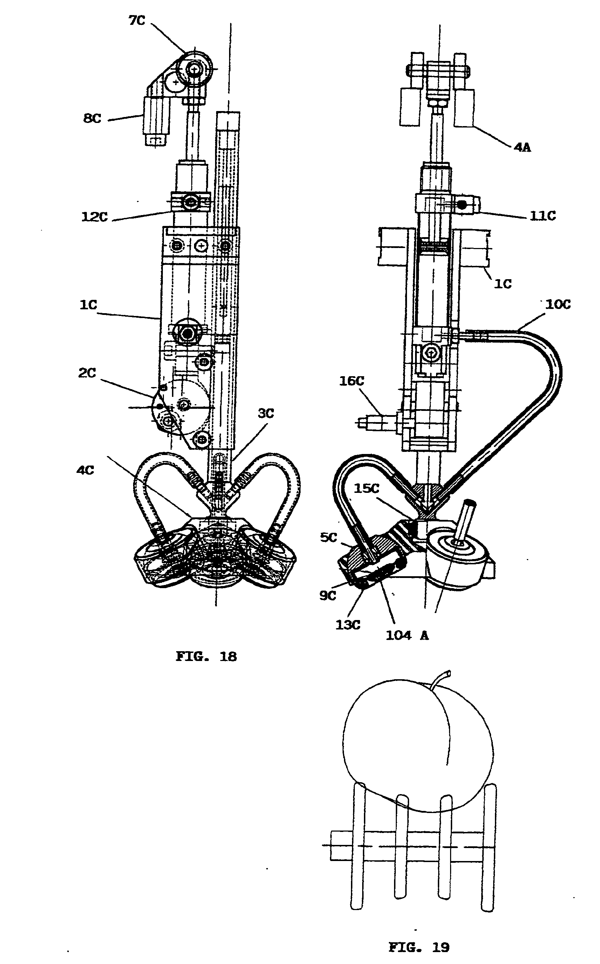

- Figure 18 is an elevational side view of some devices according to the present invention;
- Figure 19 is a front view of the same devices illustrated in Figure 18, in the initial state thereof, with the fruit represented in the condition immediately preceding the measurement;

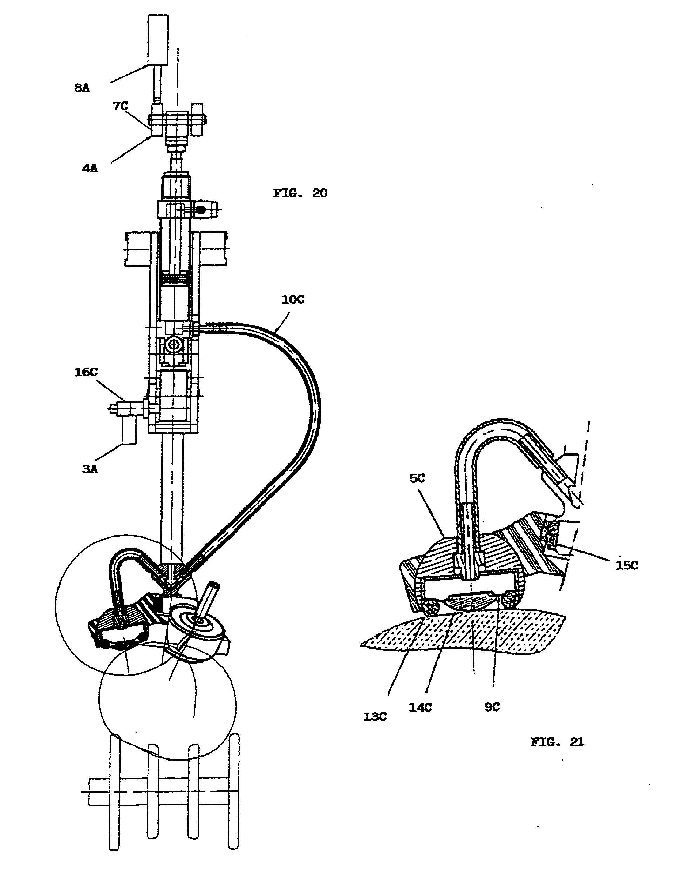

- Figure 20 is the same view as the one appearing in Figure 19, however with the devices shown in a subsequent operating state;
- Figure 21 is an enlarged cross-sectional view of a detail of Figure 20;

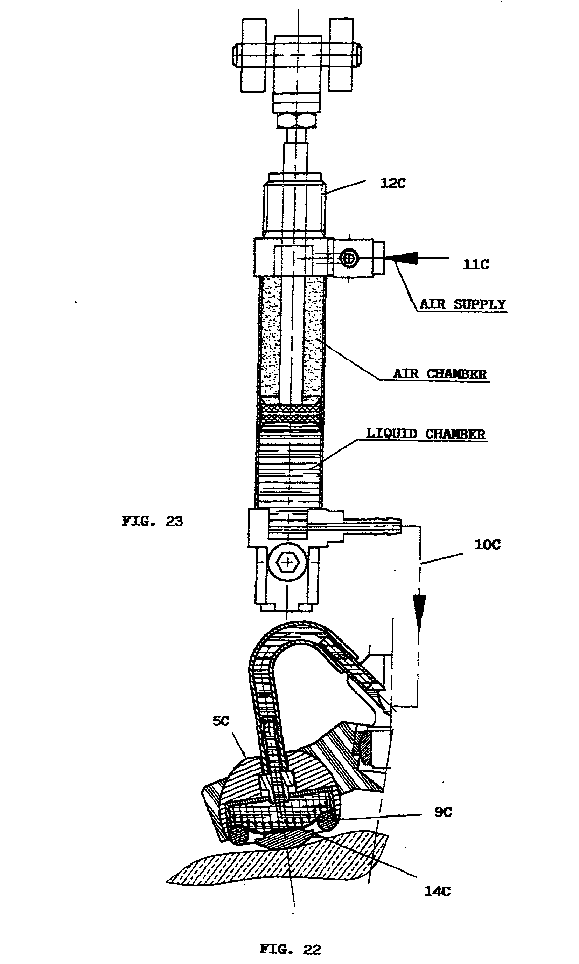

- Figure 22 is a similar enlarged view of the same detail, however as seen during the penetration phase of the sensor member;
- Figure 23 is an enlarged vertical-section view of a portion of the device illustrated in Figure 20;

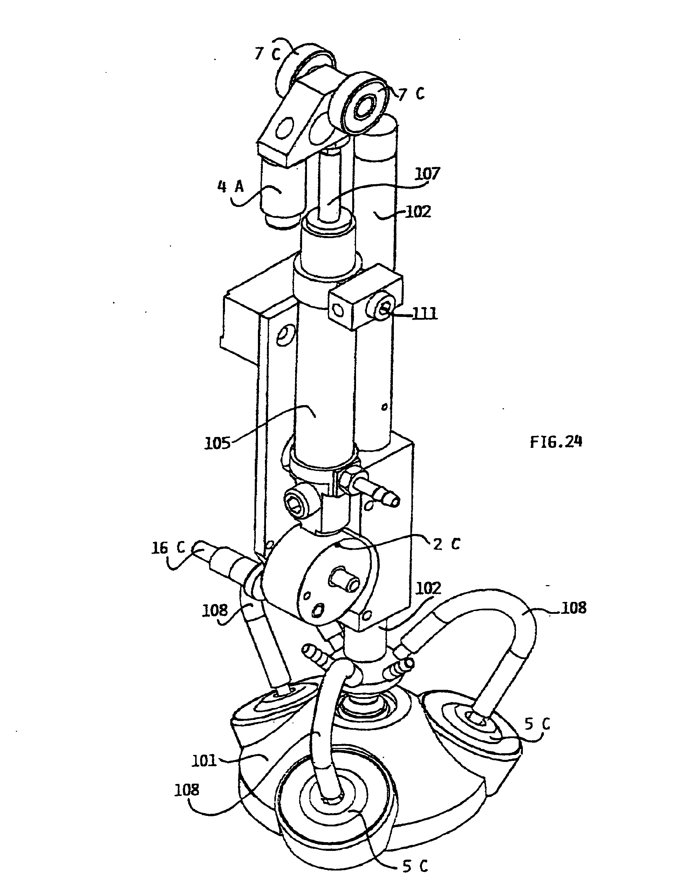

- Figure 24 is a perspective, partially cutaway view of the device illustrated in Figures 21 and 22; and

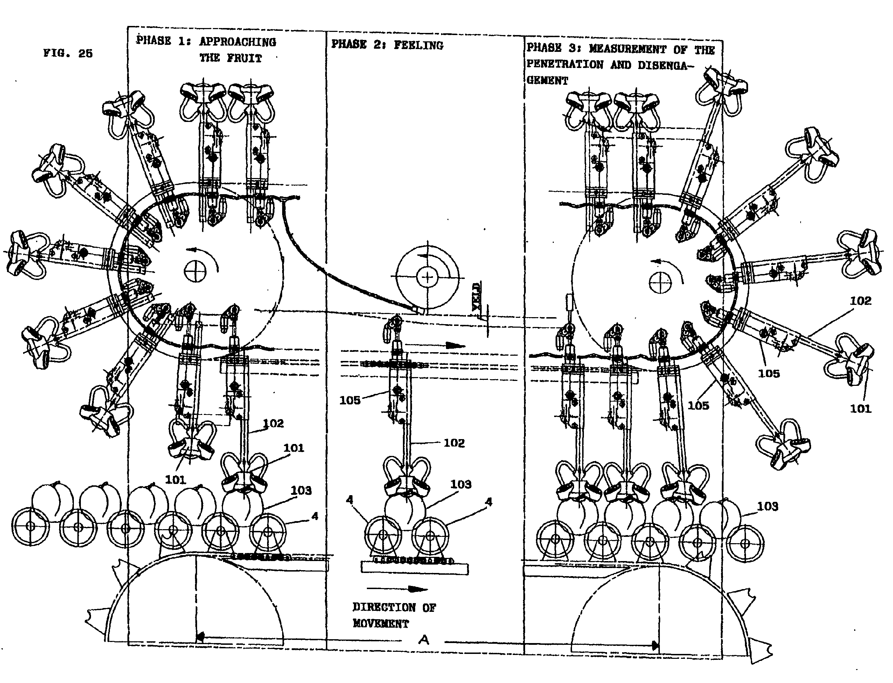

- Figure 25 is an elevational plan view of the schematical representation of the overall architecture of an apparatus and related devices according to the present invention.

With reference to Figures 15, 16 and 17, it cam be noticed that an apparatus according to an exemplified and symbolically representative embodiment of the present invention comprises:
- a mechanical hand 101,
- a guide member 102 for said mechanical hand, adapted to selectively raise and lower the same hand, as well as to be stopped automatically in the position defined by the resting position of the hand 101 on the product 103 to be measured;
- at least a membrane 104 arranged on the lower surface of said mechanical hand;
- a cylinder 105, which is divided internally by a sealing partition 106 rigidly and firmly joined to a piston or stem 107 situated on top of said partition;
- a sealed conduit 108 connecting the lower chamber 109 of said cylinder 105 with the inner volume 104B of said membrane 104;
- a conduit 111 connecting the upper chamber 110 of said cylinder 105 with the outside.

According to the invention, the volume that is determined by said inner volume 104B of said membrane 104, said conduit 108 and said lower chamber 109 is a tightly sealed volume with respect to the outside; it is further filled with an appropriate liquid, and therefore incompressible, so that said overall volume 104B + 108 + 109 is a constant one, even if it is capable of taking different shapes in accordance with the various operating states.

The operation of the above cited devices is substantially as follows:
- in a first phase (Figure 15), the guide 102 is raised and the stem 107 is in turn raised by such an extent as it is sufficient for the largest amount of liquid to be sucked in from the interior 104B of the membrane 104 and transferred into the chamber 109 of the cylinder 105, wherein no contact at all shall exist between said membrane 104 and the fruit;
- in a second phase (Figure 16), the guide 102 is lowered down to such a height as to enable the mechanical hand 101 to rest on the fruit 103 to be measured; since the latter may be irregular in its shape, to the purpose of detecting a value that is more representative than the average yielding value of the surface of the product, between said guide 102 and said mechanical hand there is provided an appropriate articulated joint, possibly a ball joint 15C, so as to allow for said mechanical hand to be able to naturally adapt, i.e. fit on the largest possible portion of the surface of the product; immediately thereafter, the guide 102 is locked automatically, with means and techniques that, being generally known in the art, shall not be described here. As soon as this state is reached, the level L reached by a reference point R located on the upper portion of the stem 107 is detected using generally known means (not shown). At the end of this phase, therefore, the mechanical hand is in a position in which it is resting on the fruit, wherein no pressure at all is exerted on the fruit 103 itself, except of course for the one due to the weight of the mechanical hand and the guide 102. It should also be noticed that the cylinder 105 does not change its position with respect to the fruit 103 under any condition;
- in the third phase (Figure 17), the upper chamber 110 is put pneumatically under pressure up to a desired value via an appropriate conduit 111 and with pneumatic pressurization means (not shown).

In these circumstances, since the mechanical hand is locked in position, the pressure in said upper chamber 110 is transferred, through the partition 106, to the lower chamber 109 and then, via the conduit 108, to said inner volume 104B of the membrane 104, which is in this way forced to expand outwardly, i.e. in the direction in which it is brought to penetrate the fruit therebelow with some appropriate fingertips 104A.

Such an expansion of the membrane produces an increase in the inner volume 104B of the same membrane and, as a result, a corresponding reduction in the volume of the lower chamber 109, owing the afore cited invariableness of the overall volumes 109 + 108 + 104B.

Owing to the lower chamber so reducing its volume, the partition 106 and, as a result, the stem 107 are caused to lower.

It has been found and demonstrated experimentally that, other conditions remaining unaltered, the lowering from a level L to a level L1, and therefore by an extent or level difference h, of a reference point whatsoever R located on said stem 107 can be exactly correlated to the penetration of the membrane and, therefore, the surface hardness of the fruit.

Reference should now be made to Figures 18 through to 22, which illustrate more realistic embodiments of an apparatus according to the present invention.

According to such Figures, some of the component parts illustrated there are similar to the component parts appearing in the preceding Figures. However, in order to avoid any possible confusion between the two groups of Figures, different reference numerals will be used.

With reference to the above cited Figures, the main phases in the operation of the apparatus according to said embodiments of the invention are described below:
- Phase 1): Approaching the fruit.
   In this phase, each penetration measuring device gets synchronized on a respective fruit which it shall measure, while keeping its mechanical hand 4C separated from said fruit until the moment is reached in which the lever 16C comes into contact with the guide 3A that acts as a cam actuating the descent of said mechanical hand.
   In this phase, before the descent of the mechanical hand is concluded, owing to a second fixed guide 4A, the fingertips 104A are forced back into a withdrawn position from which the compression described in the subsequent phase will start. Such an action occurs thanks to the pumping effect imparted to the stem of the cylinder 12C by the wheel 7C running into said fixed guide 4A, whose operation will be described in greater detail further on.
   After the mechanical hand has so completed its descent towards the fruit by fitting compliantly onto it, it is prevented from being able to perform any re-ascending motion by a locking arrangement actuated at the moment in which the cam 3A leaves go, i.e. departs.
   From this moment on, the apparatus is ready to start the subsequent compression and measurement phase.
- Phase 2): compression.
   This phase starts with the separation of the wheels 7C of the cam 4A when the stem of the cylinder 12C, which is no longer retained in its pulled-out position, is left free to move back into the same cylinder as pushed by the pressure of the gas injected into the upper volume of the cylinder by a suitable adjustable, controlled pressure source. As this will be illustrated below, to such a behaviour of the cylinder there corresponds a proportional extension of the fingertips of the mechanical hand so fitting against the fruit, and the higher is the hardness of the fruit, the more will the latter oppose such an extension. After a certain time, however, a balance is established between these opposing forces and, as a result, the penetration of the fingertips stops.
- Phase 3): measurement of the penetration and disengagement.

The rigid correspondence between the deformation of the surface of the fruit and the extension of the fingertips with the lowering of the partition and, therefore, the stem, enables the hardness of the same fruit to be assessed by measuring the extent to which the extremity of the stem of the cylinder has lowered with respect to the cam 4A which represents the reference point for such a measurement. The electronic comparator 8A detects such a yielding when the measurement device constituted by each pair of cylinders 12C and respective mechanical hands passes therebelow. It then communicates the related information to an integrated electronic control and actuation system.

When the measurement is then concluded, the mechanical hand withdraws from the fruit by raising therefrom and reversing the direction of its motion, so as to move back into its starting position and measure the hardness of a new fruit.

As this is best illustrated in the overall schematics appearing in Figure 25, as soon as said measurement device concludes the reversal of its motion and its overturn, it is automatically commanded to move back into its starting position, from which a new cycle will then start.

It should be noticed that the construction architecture and the operating mode of the component parts and members that guide the pressure and measurement devices on the moving fruit, separate them therefrom, and then move automatically back in their position at the beginning of the stretch A of Figure 25, within which there is comprised the path along which the measurement process takes place, are fully similar to those described in the afore described example, so that their description shall be omitted here for reasons of conciseness.

### COMPRESSION AND MEASUREMENT UNIT

Said measurement device, which is comprised of the assembly formed by the cylinder 12C and its respective mechanical hand, constitutes the element that is capable of transducing the hardness of the fruit into a measurable displacement of the extremity of a target. Three distinct parts are contained therewithin, i.e.:
- the pressure and penetration unit translating on a linear sliding guide;
- the pumping unit carrying said target for measuring the yielding extent of the fruit;
- the locking unit for stopping the translating motion of said pressure unit; this is constituted by a mechanical hand 101 mounted via a ball joint 15C on the guide 102, which is allowed to slide on an appropriate guide of the framework. Such a mechanical hand is provided with an arrangement of retractable and swivelling fingertips. Each such fingertip is associated to a hollow spherical bulb 5C, free of rotating about its own centre, whose cavity is tightly sealed by a flexible membrane 104A.

At the centre of said membrane there is located a respective fingertip 104, while an orienting ring 13C is inserted peripherally. The cavity of each fingertip is filled with liquid and is connected via flexible tubes to the cavities of the other fingertips and the whole is connected via the tube 10C to the cylinder 12C.

The compression/penetration unit is provided with all degrees of freedom as necessary and adequate to ensure that, when it is brought into contact with a curved surface whatsoever, each fingertip tends spontaneously to enter into contact with said surface, while assuming an axis which is orthogonal to the same surface in the zone of contact thereof.

The pumping unit is constituted by a double-acting cylinder 12C, of which one of the two chambers separated by the piston is full of liquid and is connected via the flexible tube 10C to the fingertips, whereas the other chamber is pressurized in a controlled manner by a gas admitted thereinto through the fitting 10C. On the upper extremity of the stem of said cylinder there are positioned the wheels that constitute both the reference for the measurement of the yielding extent of the product being tested and the elements sliding on the cams 4A.

These wheels are kept oriented correctly by an appropriate steering member sliding between the cams 4A.

In the compression phase, the liquid contained in the cylinder is transferred into the fingertips in a quantity that is proportional to the extent by which they come out from the swivelling bulbs 5C, wherein said fingertips will be pushed against the surface of the fruit or product being measured with a force that is proportional to the pressure existing in the cylinder itself as determined by the gas admitted thereinto from said fitting 10C.

Owing to the incompressibility of the liquid, the stroke of the piston of said cylinder (which is quantifiable from the outside through the measurement of the stroke of the stem) is indicative of the extent of yielding of the surface of the fruit submitted to said force of penetration.

Something must anyway be stressed in this connection: it has been stated that the fruit or vegetable product to be measured is first arranged on a respective appropriate cradle; to the purpose of being able to perform a measurement that is as complete, indicative and reliable as possible as far as the actual condition of the whole product, the need arises for the largest possible portion of the surface to be pressed and the related yield to be measured.

It is therefore advantageous for the pressure of the various fingertips of the mechanical hand to be used for measuring the overall yield of the product. In fact, if the related cradle is made in such a manner as to be substantially rigid and undeformable, as this actually appears to be adequate, the pressure exerted by the mechanical hand will give rise to respective obvious deformations both in the zone of contact of said fingertips and the zone in which the product rests on the respective cradle.

It is therefore quite obvious that these deformations add to each other and therefore allow for a greater excursion of the fingertips of the mechanical hand; such a lowering thereof is therefore well representative of the yield of distinct portions of the surface of the product to be measured and, as a result, the measurement thereof becomes much more reliable and representative.

Such a particular feature of the transducer enables the hardness of the fruit, to which the degree of ripening thereof is associated, to be measured in a substantially complete manner.

The sliding motion stopping unit is constituted by the eccentric-type cam 2C with the related actuation lever 16C. This cam tends to spontaneously move into contact with the guide 102 and, by interfering with said guide, prevents it from being able to slide; conversely, if it is caused to rotate by means of its own lever 16C (by the action of appropriate cams that are not shown in the Figures), said stopping and sliding unit moves away from the stem itself, thereby enabling it to slide freely.

In an operation cycle, such a cam is actuated twice, i.e. a first time when, in the fruit approaching phase, the mechanical hand is desired to be left free to fall and settle on said fruit before it remains locked in the reached position, and a second time when, upon disengaging the fruit, the device is reset, i.e. brought to its initial state and position in view of preparing it for a new cycle.

## Claims

1. Apparatus for the non-destructive measurement of individual non-uniformly shaped and sized soft products (3), such as vegetable products or the like, comprising:
- a plurality of conveying means adapted to support and carry said individual products along a conveying path from an inlet station to an outlet station of said products, said means being preferably in the form of mutually aligned cradles (4) adapted to support respective said products when placed therein from above in an orderly sequence and transported individually in a definite, substantially horizontal direction coinciding with the alignment of said product accommodating sectors;
- a first driving means (2) engaging said conveying means to which said product accommodating sectors are connected in an orderly arrangement so as to be caused to move synchronously;
- moving means (24) adapted to move into contact with said respective individual products on the opposite side with respect to the one which is in contact with said respective conveying means;
- means (25) adapted to act on said moving means so as to exert an initial controlled pressure on said individual products (3) in such a manner as to establish an initial position datum-point,
**characterized in that**:
- there is defined a measurement stretch (A) comprised in said conveying path of the individual products and provided with an initial position and a final position (34):
- said moving means (24) are adapted so as to be capable of coming automatically into contact with said respective individual products when these pass through said initial position, and are also adapted to be automatically locked in the resting position that they so assume on said respective individual products when passing through said initial position;
- said moving means are adapted to move synchronically with said cradles and, therefore, with said respective individual products (3) along said measurement stretch (A);
- there are provided guiding means (16) and support means (15) adapted, as they move along said measurement stretch, to progressively approach said moving means and said cradles in such a manner as to cause said individual products to undergo a gradually increasing pressure;
- in said final position (34) said moving means (24) are so arranged as to be able to be separated automatically from said respective individual products, and there are provided further measurement and control means (33, 35) adapted to measure and record the deviation between the initial distance and the final distance, ie. across the measurement stretch, of each moving means (24) with respect to the respective cradle (4) due to the action of said controllable pressure.

2. Apparatus according to claim 1, **characterized in that** said moving means (24) are driven to move synchronically with said respective cradles by means of a second driving means that is preferably constituted by a second closed-loop chain (22) engaged by two distinct gearwheels (12, 13) driven in a synchronous manner with respect to each other, in which said moving means in said measurement stretch are adapted to arrange themselves in a position above a respective one of said cradles (4).

3. Apparatus according to claim 1 or 2, **characterized in that** said conveyance path comprises:
- a first ramp (15A) to accede up to a sliding plane (15);
- contact means, preferably provided with wheels (6), adapted to support and connect said cradles to said ramps and said sliding plane;
- a parallelogram-shaped structure (17) adapted to connect each one of said cradles and the respective engaging means to said first driving means (2).

4. Apparatus according to claim 3, **characterized in that** said first driving means comprises a chain moving along a closed-loop path arranged on a vertical plane, in which said chain is supported and driven rotatably by means of two appropriate gearwheels (10, 11) provided on the outside of said measurement stretch (A) on the opposite sides thereof.

5. Apparatus according to any of the preceding claims, **characterized in that** said progressively increasing pressure is obtained by means of a slightly converging profile between said guide means (16) and said support means (15) of the respective cradles in said measurement stretch.

6. Apparatus according to any of the preceding claims, **characterized in that** said cradles comprise at least three preferably parallel support elements (44) oriented towards said individual product (3).

7. Apparatus according to any of the preceding claims, **characterized in that** said moving means (24) comprise a plurality of rods (45) which are preferably parallel and oriented coaxially with respect to said three support elements

8. Apparatus according to any of the preceding claims, **characterized in that** said guide means comprise a longitudinal structure (16) provided with a plurality of continuous elastic elements (30) that are substantially as long as said adjustable-resistance structure, on which there is adapted to press and slide a contact member (27) that is firmly joined to a respective moving means (24).

9. Apparatus according to claim 8, **characterized in that** said longitudinal structure is hollow, and said elastic elements (30) are constituted by respective tubular elements inflated at an adjustable pressure.

10. Apparatus according to any of the preceding claims, **characterized in that** said guide means (16) and said support means (15) may be arranged in a alternately overlying manner.

11. Apparatus according to any of the preceding claims 7 to 10, **characterized in that** said elastic elements are provided in a parallel pair (30, 30A), and that said contact member is constituted by a middle wheel (23A) that is symmetrical to said elastic elements.

12. Apparatus according to the preamble of claim 1, **characterized in that**:
- there is defined a measurement stretch, comprised in a lower portion (A) of said conveyance path, and provided with an initial position and a final position (34);
- said moving means are adapted so as to be capable of being brought automatically into contact with said respective individual products (103) when these pass through said initial position, and are adapted to be automatically locked in the resting position that they so assume on said respective individual products when they pass through said initial position;
- said moving means are adapted to move synchronically with said cradles and, therefore, with said respective individual products (103) along said measurement stretch, and comprise:
- a mechanical hand (101),
- a guide (102) and support for said mechanical hand,
- a cylinder (105) subdivided into an upper volume (110) and a lower volume (109) by a sealing partition (106) adapted to slide inside said cylinder (105),
- a first conduit (108) connecting said lower volume (109) with said moving means,
- a second conduit (111) connecting said upper volume (110) with an appropriate source of pneumatic pressure,
- a stem (107) joined to said partition (106) on the top thereof.

13. Apparatus according to claim 12, **characterized in that** said mechanical hand (101) comprises a plurality of swivelling "fingertips" (104A) protruding from a flexible membrane (104) and delimiting a respective inner volume (104B) and associated to a respective hollow spherical bulb (5C).

14. Apparatus according to claim 13, **characterized in that** said inner volume (104B), said first conduit (108) and said lower volume (109) altogether constitute a single volume made up of freely communicating vessels that are filled with a liquid, so that the related overall volume remains constant.

15. Apparatus according to claim 14, **characterized in that** said swivelling fingertips (104A) are housed within respective orienting rings (13C).

16. Apparatus according to any of the preceding claims 12 to 15, **characterized in that** it is provided with means (8A) adapted to measure the variation (h) between distinct levels (L1, L) of said stem (107) as these are assumed by said stem (107) between two pre-defined moments.

17. Apparatus according to claim 16, **characterized in that** said vegetable products (103) are brought into movement by first driving means comprising a chain moving along a closed-loop path arranged on a vertical plane, and adapted to slide along said measurement stretch (A), and that said cylinder (105), said guide (102) along with the members and means associated therewith are driven so as to move synchronously with said respective cradles by means of a second driving means that is preferably constituted by a second closed-loop chain moving synchronically, and that said moving means in said measurement stretch arrange themselves in a position that is substantially above a respective one of said cradles (4).

18. Apparatus according to any of the preceding claims 14 to 17, **characterized in that** it is provided with means adapted to selectively cause said fingertips to return into a retracted position, as well as further means adapted to prevent said mechanical hand from being able to perform any re-ascending motion after it has completed its descent towards the respective product to be measured and has come to rest by gravity thereonto.

19. Apparatus according to any of the preceding claims 12 to 18, **characterized in that** said guide (102) supporting said mechanical hand (101) is provided with a ball joint (15C) adapted to allow for the rotation and the automatic adaptation of the position of said mechanical hand at the extremity of said guide.

20. Method for the measurement of the yielding extent of a portion of the surface of a vegetable or fruit product, **characterized in that** it comprises at least a first phase in which a measuring device approaches the product to be measured, a second phase in which said product is submitted to a controlled compression so as to cause said measuring device to partially penetrate the surface of the product, and a third phase in which said penetration is measured, in which:
- in said first phase, said measuring device gets synchronized on a respective product, whole keeping separated from it and arranged vertically above it until a pre-defined position is eventually reached;
- in said second phase, a part of said measuring deice is pressed with a controlled force against at least a portion of the surface of the product so as to be able to partially penetrate it, by synchronically moving said measuring device and the respective product in an uniform and continuous way, parallel along a same stretch "A" and therefore for a pre-definite time-length,
- in said third phase, the extent by which said measuring device has penetrate the product is automatically measured and the result of such a measurement is processed so as to define a characterization of the respective product and rate it accordingly.

## Patentansprüche

1. Vorrichtung zum zerstörungsfreien Messen einzelner Weichprodukte (3) nicht einheitlicher Form und Größe, wie etwa Gemüseprodukten oder dergleichen, enthaltend:
- eine Vielzahl von Fördereinrichtungen, die dazu eingerichtet sind, die einzelnen Produkte entlang eines Förderweges von einer Einlassstation zu einer Auslassstation der Produkte zu halten und zu transportieren, wobei die Einrichtungen vorzugsweise die Gestalt von wechselseitig angeordneten Gabeln (4) haben, die dazu eingerichtet sind, die jeweiligen Produkte zu halten, wenn sie in diesen in einer geordneten Abfolge von oben plaziert und einzeln in einer festgelegten, im wesentlichen horizontalen, Richtung transportiert werden, die mit der Ausrichtung der Produktaufnahmeabschnitte übereinstimmt;
- eine erste Antriebseinrichtung (2), die mit den Fördereinrichtungen in Eingriff steht, mit denen die Produktaufnahmeabschnitte in einer geordneten Anordnung verbunden sind, so dass eine synchrone Bewegung bewirkt wird;
- Bewegungseinrichtungen (24), die dazu eingerichtet sind, sich in einen Kontakt mit den jeweils einzelnen Produkten auf der gegenüberliegenden Seite im Bezug auf jene zu bewegen, die mit der jeweiligen Fördereinrichtung in Kontakt steht;
- eine Einrichtung (25), die dazu eingerichtet ist, auf die Bewegungseinrichtung derart einzuwirken, dass sie einen gesteuerten Ausgangsdruck auf die einzelnen Produkte (3) so ausübt, dass ein Ausgangspositions-Bezugspunkt erzeugt wird,
**dadurch gekennzeichnet, dass**:
- es eine Messtrecke (A) gibt, die in dem Förderweg der einzelnen Produkte enthalten ist und über eine Ausgangsposition sowie eine Endposition (34) verfügt;
- die Bewegungseinrichtungen (24) so eingerichtet sind, dass sie automatisch mit den jeweiligen einzelnen Produkten in Kontakt gelangen können, wenn diese die Ausgangsposition durchlaufen, und zudem dazu eingerichtet sind automatisch in der Ruhestellung arretiert zu werden, die sie an den jeweiligen einzelnen Produkten einnehmen, wenn sie die Ausgangsposition durchlaufen;
- die Bewegungseinrichtungen dazu eingerichtet sind, sich synchron mit den Gabeln und somit mit den jeweiligen einzelnen Produkten (3) entlang der Messstrecke (A) zu bewegen;
- Führungseinrichtungen (16) und Halteeinrichtungen (15) vorgesehen sind, die dazu eingerichtet sind, während sie sich entlang der Messstrecke bewegen, allmählich den Bewegungseinrichtungen und den Gabeln derart anzunähern, dass bewirkt wird, dass einzelnen Produkte einem allmählich zunehmenden Druck ausgesetzt werden; und
- in der Endposition (34) die Bewegungseinrichtungen (24) derart angeordnet sind, dass sie automatisch von den jeweiligen einzelnen Produkten getrennt werden können, und weitere Mess- sowie Steuereinrichtungen (33, 35) vorgesehen sind, die dazu eingerichtet sind, die Abweichung zwischen der Ausgangsdistanz und der Enddistanz, d.h. über die Messstrecke, jeder Bewegungseinrichtung (24) im Bezug auf die entsprechende Gabel (4) infolge der Einwirkung des steuerbaren Drucks zu messen und aufzuzeichnen.

2. Vorrichtung nach Anspruch 1, **dadurch gekennzeichnet, dass** die Bewegungseinrichtungen (24) derart, dass sie sich synchron mit entsprechenden Gabeln bewegen, mit Hilfe einer zweiten Antriebseinrichtung angetrieben werden, die vorzugsweise aus einer zweiten Endloskette (22) besteht, die mit zwei getrennten Zahnrädern (12, 13) in Eingriff steht, die im Bezug zueinander synchron angetrieben werden, wobei die Bewegungseinrichtungen in der Messstrecke dazu eingerichtet sind, sich selbst in einer Position über einer entsprechenden der Gabeln (4) anzuordnen.

3. Vorrichtung nach Anspruch 1 oder 2, **dadurch gekennzeichnet, dass** der Förderweg enthält:
- eine erste Rampe (15A), die aufwärts zu einer Gleitebene (15) ansteigt;
- eine Kontakteinrichtung, die vorzugsweise mit Rädern (6) versehen und dazu eingerichtet ist, die Gabeln zu halten und sie mit der Rampe und der Gleitfläche zu verbinden; und
- eine parallelogrammförmige Struktur (17), die dazu eingerichtet ist, jede der Gabeln und der entsprechenden Eingriffseinrichtungen mit der ersten Antriebseinrichtung (2) zu verbinden.

4. Vorrichtung nach Anspruch 3, **dadurch gekennzeichnet, dass** die erste Antriebseinrichtung eine Kette enthält, die sich entlang eines Endlosweges bewegt, der auf einer vertikalen Ebene angeordnet ist, wobei die Kette von zwei geeigneten Zahnrädern (10, 11) gehalten und drehend angetrieben wird, die außerhalb der Messstrecke (A) auf gegenüberliegenden Seiten derselben vorgesehen sind.

5. Vorrichtung nach einem der vorhergehenden Ansprüche, **dadurch gekennzeichnet, dass** der allmählich ansteigende Druck mit Hilfe eines sich leicht verjüngenden Profils zwischen der Führungseinrichtung (16) und der Halteeinrichtung (15) der entsprechenden Gabeln in der Messstrecke erzeugt wird.

6. Vorrichtung nach einem der vorhergehenden Ansprüche, **dadurch gekennzeichnet, dass** die Gabeln wenigstens drei, vorzugsweise parallele Halteelemente (44) enthalten, die zu jedem einzelnen Produkt (3) hin ausgerichtet sind.

7. Vorrichtung nach einem der vorhergehenden Ansprüche, **dadurch gekennzeichnet, dass** die Bewegungseinrichtungen (24) eine Vielzahl von Stäben (45) enthalten, die vorzugsweise parallel und koaxial im Bezug auf die drei Halteelemente ausgerichtet sind.

8. Vorrichtung nach einem der vorhergehenden Ansprüche, **dadurch gekennzeichnet, dass** die Führungseinrichtungen eine längliche Struktur (16) enthalten, die mit einer Vielzahl kontinuierlicher elastischer Elemente (30) ausgestattet ist, die im wesentlichen so lang sind wie die Anordnung mit einstellbarem Widerstand, auf der ein Kontaktelement (27) eingerichtet ist, das einen Druck ausüben und gleiten kann und fest mit einer entsprechenden Bewegungseinrichtung (24) verbunden ist.

9. Vorrichtung nach Anspruch 8, **dadurch gekennzeichnet, dass** die längliche Struktur hohl ist und die elastischen Elemente (30) aus entsprechenden röhrenförmigen Elementen bestehen, die mit einem einstellbaren Druck aufgepumpt werden.

10. Vorrichtung nach einem der vorhergehenden Ansprüche, **dadurch gekennzeichnet, dass** die Führungseinrichtungen (16) und die Halteeinrichtungen (17) alternierend überlagernd angeordnet sein können.

11. Vorrichtung nach einem der vorhergehenden Ansprüche 7 bis 10, **dadurch gekennzeichnet, dass** die elastischen Elemente in einem parallelen Paar (30, 30A) vorgesehen sind und das Kontaktelement aus einem Mittelrad (23A) besteht, das zu den elastischen Elementen symmetrisch ist.

12. Vorrichtung nach dem Oberbegriff von Anspruch 1, **dadurch gekennzeichnet, dass**:
- eine Messstrecke vorgesehen ist, die in einem unteren Abschnitt (A) des Förderweges enthalten ist und über eine Ausgangsposition sowie eine Endposition (34) verfügt;
- die Bewegungseinrichtungen derart eingerichtet sind, dass sie in der Lage sind, automatisch mit den jeweiligen einzelnen Produkten (103) in Kontakt gebracht zu werden, wenn diese die Ausgangsposition durchlaufen, und dazu eingerichtet sind, automatisch in der Ruhestellung arretiert zu werden, die sie an den jeweiligen einzelnen Produkten einnehmen, wenn sie die Ausgangsposition durchlaufen; und
- die Bewegungseinrichtungen dazu eingerichtet sind, sich synchron mit den Gabeln und somit mit den jeweiligen einzelnen Produkten (103) entlang der Messstrecke zu bewegen, und enthalten
- einen mechanischen Greifer (101),
- eine Führung (102) und eine Halteeinrichtung für den mechanischen Greifer,
- einen Zylinder (105), der in ein oberes Volumen (110) und ein unteres Volumen (109) durch einen Dichtungsabschnitt (106) unterteilt ist, der dazu eingerichtet ist, in dem Zylinder (105) zu gleiten,
- eine erste Leitung (108), die das untere Volumen (109) mit der Bewegungseinrichtung verbindet,
- eine zweite Leitung (111), die das obere Volumen (110) mit einer geeigneten Quelle eines pneumatischen Drucks verbindet, und
- einen Schaft (107) der mit der Trenneinrichtung (106) an deren Oberseite verbunden ist.

13. Vorrichtung nach Anspruch 12, **dadurch gekennzeichnet, dass** der mechanische Greifer (101) eine Vielzahl schwenkender "Fingerspitzen" (1 04A) enthält, die von einer flexiblen Membran (104) hervorragen und ein entsprechendes Innenvolumen (104B) begrenzen und einem entsprechenden hohlen, kogelförmigen Kolben (5C) zugeordnet sind.

14. Vorrichtung nach Anspruch 13, **dadurch gekennzeichnet, dass** das Innenvolumen (104B), die erste Leitung (108) und das untere Volumen (109) zusammen ein einzelnes Volumen bilden, das aus frei kommunizierenden Behältern besteht, die mit einer Flüssigkeit gefüllt sind, so dass das entsprechende Gesamtvolumen konstant bleibt.

15. Vorrichtung nach Anspruch 14, **dadurch gekennzeichnet, dass** sich die schwenkenden Fingerspitzen (104A) in entsprechenden Einstellringen (13C) befinden.

16. Vorrichtung nach einem der vorhergehenden Ansprüche 12 bis 15, **dadurch gekennzeichnet, dass** sie mit einer Einrichtung (8A) versehen ist, die dazu eingerichtet ist, die Änderung (h) zwischen bestimmten Höhen (L1, L) des Schaftes zu messen, wenn diese von dem Schaft (107) zwischen zwei vorbestimmten Zeitpunkten eingenommen werden.

17. Vorrichtung nach Anspruch 16, **dadurch gekennzeichnet, dass** die Gemüseprodukte (103) von der ersten Antriebseinrichtung in Bewegung gebracht werden, die eine Kette enthält, die sich entlang eines Endlosweges bewegt, der auf einer vertikalen Ebene angeordnet ist, und die dazu eingerichtet ist, entlang der Messstrecke (A) zu gleiten, und dass der Zylinder (105) sowie die Führung (102) zusammen mit den Elementen und den damit zugeordneten Einrichtungen derart, dass sie sich synchron mit entsprechenden Gabeln bewegen, von einer zweiten Antriebseinrichtung angetrieben werden, die vorzugsweise aus einer zweiten Endloskette besteht, die sich synchron bewegt, und dass sich die Bewegungseinrichtungen in der Messstrecke selbst in einer Position ausrichten, die im wesentlichen über einer entsprechenden der Gabeln (4) liegt.

18. Vorrichtung nach einem der vorhergehenden Ansprüche 14 bis 17, **dadurch gekennzeichnet, dass** sie mit Einrichtungen, die dazu eingerichtet sind, wahlweise zu bewirken, dass die Fingerspitzen in eine zurückgezogene Stellung zurückkehren, wie auch weiteren Einrichtungen ausgestattet ist, die dazu eingerichtet sind zu verhindern, dass der mechanische Greifer in der Lage ist, eine erneute Hebebewegung auszuführen, nachdem er seine Absenkbewegung hin zum jeweiligen zu messenden Produkt ausgeführt hat und auf diesem infolge der Schwerkraft ruht.

19. Vorrichtung nach einem der vorhergehenden Ansprüche 12 bis 18, **dadurch gekennzeichnet, dass** die Führung (102), die den mechanischen Greifer (101) hält, mit einem Kugelgelenk (15C) ausgestattet ist, das dazu eingerichtet ist, die Drehung und die automatische Anpassung der Position des mechanischen Greifers am äuβersten Ende der Führung zu gestatten.

20. Verfahren zum Messen des Nachgiebigkeitsumfangs eines Oberflächenabschnittes eines Gemüse- oder Obstproduktes, **dadurch gekennzeichnet**, das es wenigstens eine erste Phase, in der sich eine Messvorrichtung dem zu messenden Produkt nähert, und eine zweite Phase, in der das Produkt einem gesteuerten Druck ausgesetzt wird, um zu bewirken, dass die Messvorrichtung teilweise in die Oberfläche des Produktes eindringt, sowie eine dritte Phase umfasst, bei der das Eindringen gemessen wird, wobei:
- in der ersten Phase die Messvorrichtung mit einem entsprechenden Produkt synchronisiert wird, sie insgesamt von diesem getrennt bleibt und vertikal über diesem angeordnet ist, bis schließlich eine vorbestimmte Position erreicht ist;
- in der zweiten Phase ein Teil der Messvorrichtung mit einer gesteuerten Kraft gegen wenigstens einen Abschnitt der Oberfläche des Produktes gedrückt wird, um so in der Lage zu sein, teilweise in dasselbe einzudringen, indem die Messvorrichtung und das jeweilige Produkt in einer einheitlichen und kontinuierlichen Art und Weise parallel entlang der Strecke "A" und somit eine vordefinierte Zeitdauer bewegt werden, und
- in der dritten Phase der Umfang, in dem die Messvorrichtung in das Produkt eingedrungen ist, automatisch gemessen wird und das Ergebnis einer derartigen Messung verarbeitet wird, um so eine Charakterisierung des jeweiligen Produktes zu definieren und es dementsprechend zu klassifizieren.

## Revendications

1. Appareil pour la mesure non destructive de produits (3) souples individuels, de forme et de taille non uniformes, tels que des produits de légumes ou analogues, comprenant :
- une pluralité de moyens de convoyage adaptés pour supporter et transporter lesdits produits individuels le long d'un chemin de convoyage depuis une station d'entrée jusqu'à une station de sortie desdits produits, lesdits moyens étant préférablement sous la forme de berceaux (4) alignés mutuellement, adaptés pour supporter lesdits produits respectifs lorsqu'ils y sont placés depuis le dessus dans une séquence ordonnée et sont transportés individuellement dans une direction définie, substantiellement horizontale, coïncidant avec l'alignement desdits secteurs de réception de produits;
- un premier moyen d'entraînement (2) en prise avec lesdits moyens de convoyage, auquel sont reliés lesdits secteurs de réception de produits, dans un agencement ordonné de manière à être déplacés de façon synchrone ;
- des moyens de déplacement (24) adaptés pour se déplacer en contact avec lesdits produits individuels respectifs, sur le côté opposé par rapport à celui qui est en contact avec lesdits moyens de convoyage respectifs ;
- des moyens (25) adaptés pour agir sur lesdits moyens de déplacement de manière à exercer une pression initiale commandée sur lesdits produits individuels (3) de telle manière à établir un point de donnée de position initiale,
**caractérisé en ce que**
- il est défini une étendue de mesure (A) comprise dans ledit chemin de convoyage des produits individuels et pourvue d'une position initiale et d'une position finale (34);
- lesdits moyens de déplacement (24) sont adaptés de façon à être capables de venir automatiquement en contact avec lesdits produits individuels respectifs quand ceux-ci passent au droit de ladite position initiale, et sont aussi adaptés pour être automatiquement verrouillés dans la position de repos qu'ils assurent ainsi sur lesdits produits individuels respectifs en passant au droit de ladite position initiale ;
- lesdits moyens de déplacement sont adaptés pour se déplacer de façon synchrone avec lesdits berceaux et, par conséquent, avec lesdits produits individuels respectifs (3) le long de ladite étendue de mesure (A) ;
- il est prévu des moyens de guidage (16) et des moyens de support (15) adaptés, lorsqu'ils se déplacent le long de ladite étendue de mesure, pour s'approcher progressivement desdits moyens de déplacement et desdits berceaux de manière à forcer lesdits produits individuels à subir une pression graduellement croissante ;
- dans ladite position finale (34), lesdits moyens de déplacement (24) sont agencés de façon à être capables d'être automatiquement séparés desdits produits individuels respectifs, et il est prévu des moyens supplémentaires de commande et de mesure (33, 35) adaptés pour mesurer et enregistrer la déviation entre le distance initiale et la distance finale, à savoir à travers l'étendue de mesure, de chaque moyen de déplacement (24) par rapport au berceau respectif (4), du fait de l'action de ladite pression pouvant être commandée.

2. Appareil selon la revendication 1, **caractérisé en ce que** lesdits moyens de déplacement (24) sont entraînés de façon à se déplacer de façon synchrone avec lesdits berceaux respectifs au moyen d'un second moyen d'entraînement qui est de préférence constitué par une seconde chaîne en boucle fermée (22) en prise avec deux roues dentées distinctes (12, 13) entraînées de façon synchrone l'une par rapport à l'autre, dans lequel lesdits moyens de déplacement dans ladite étendue de mesure sont adaptés pour se placer eux-mêmes dans une position au-dessus de l'un respectif desdits berceaux (4).

3. Appareil selon la revendication 1 ou 2, **caractérisé en ce que** ledit chemin de convoyage comprend
- une première rampe (15A) pour s'éleverjusqu'à un plan coulissant (15) ;
- des moyens de contact, de préférence pourvus de roues (6), adaptés pour supporter et relier lesdits berceaux auxdites rampes et audit plan coulissant ;
- une structure en forme de parallélogramme (17) adaptée pour relier chacun desdits berceaux et les moyens de prise respectifs audit premier moyen d'entraînement (2).

4. Appareil selon la revendication 3, **caractérisé en ce que** ledit premier moyen d'entraînement comprend une chaîne mobile le long d'un chemin en boucle fermée agencé sur un plan vertical, dans lequel ladite chaîne est supportée et entraînée en rotation au moyen de deux roues dentées (10, 11) appropriées, prévues sur l'extérieur de ladite étendue de mesure (A) sur ses côtés opposés.

5. Appareil selon l'une quelconque des revendications précédentes, **caractérisé en ce que** ladite pression graduellement croissante est obtenu au moyen d'un profil légèrement convergent entre lesdits moyens de guidage (16) et lesdits moyens de support (15) des berceaux respectifs dans ladite étendue de mesure.

6. Appareil selon l'une quelconque des revendications précédentes, **caractérisé en ce que** lesdits berceaux comprennent au moins trois éléments de support (44) de préférence parallèles, orientés vers lesdits produits individuels (3)

7. Appareil selon l'une quelconque des revendications précédentes, **caractérisé en ce que** lesdits moyens de déplacement (24) comprennent une pluralité de tiges (45) qui sont de préférence parallèles et orientées de manière coaxiale par rapport auxdits trois éléments de support.

8. Appareil selon l'une quelconque des revendications précédentes, **caractérisé en ce que** lesdits moyens de guidage comprennent une structure longitudinale (16) pourvue d'une pluralité d'éléments élastiques continus (30) qui sont sensiblement aussi longs que ladite structure de résistance ajustable, sur laquelle il est prévu un élément de contact (27) adapté pour presser et coulisser, qui est fermement lié à un moyen de déplacement respectif (24).

9. Appareil selon la revendication 8, **caractérisé en ce que** ladite structure longitudinale est creuse, et lesdits éléments élastiques (30) sont constitués par des éléments tubulaires respectifs gonflés à une pression ajustable.

10. Appareil selon l'une quelconque des revendications précédentes, **caractérisé en ce que** lesdits moyens de guidage (16) et lesdits moyens de support (15) peuvent être agencés en recouvrement alterné.

11. Appareil selon l'une quelconque des revendications précédentes 7 à 10, **caractérisé en ce que** lesdits éléments élastiques sont prévus selon une paire parallèle (30, 30A), et ledit élément de contact est constitué par une roue médiane (23A) qui est symétrique par rapport auxdits éléments élastiques.

12. Appareil selon le préambule de la revendication 1, **caractérisé en ce que** :
- il est défini une étendue de mesure, comprise dans une portion inférieure (A) dudit chemin de convoyage, et pourvue d'une position initiale et d'une position finale (34) ;
- lesdits moyens de déplacement sont adaptés de façon à être capables d'être amenés automatiquement en contact avec lesdits produits individuels respectifs (103) quand ceux-ci passent au droit de ladite position initiale, et sont adaptés pour être automatiquement verrouillés dans la position de repos qu'ils assurent ainsi sur lesdits produits individuels respectifs en passant au droit de ladite position initiale ;
- lesdits moyens de déplacement sont adaptés pour se déplacer de façon synchrone avec lesdits berceaux et, par conséquent, avec lesdits produits individuels respectifs (103) le long de ladite étendue de mesure, et comprennent :
- une main mécanique (101),
- un guide (102) et support pour ladite main mécanique,
- un cylindre (105) subdivisé en un volume supérieur (110) et un volume inférieur (109) par une garniture d'étanchéité (106) adaptée pour coulisser à l'intérieur dudit cylindre (105),
- un premier conduit (108) reliant ledit volume inférieur (109) et les moyens mobiles,
- un second conduit (111) reliant ledit volume supérieur (110) avec une source appropriée de pression pneumatique,
- une tige (107) reliée à la garniture (106) sur le sommet de cette dernière.

13. Appareil selon l'une la revendication 12, **caractérisé en ce que** ladite main mécanique (101) comprend une pluralité de « bouts de doigt » pivotants (104A) faisant saillie depuis une membrane flexible (104) et délimitant un volume intérieur respectif (104B) et associés à un bulbe sphérique creux respectif (5C).

14. Appareil selon la revendication 13, **caractérisé en ce que** ledit volume intérieur (104B), ledit premier conduit (108) et ledit volume inférieur (109) constituent ensemble un unique volume fait de réservoirs en communication libre qui sont remplis d'un liquide, de sorte que le volume général considéré reste constant.

15. Appareil selon la revendication 14, **caractérisé en ce que** lesdits bouts de doigt pivotants (104A) sont logés à l'intérieur de bagues d'orientation respectives (13C).

16. Appareil selon l'une quelconque des revendications précédentes 12 à 15, **caractérisé en ce qu'**il est pourvu de moyens (8A) adaptés pour mesurer la variation (h) entre des niveaux distincts (L1, L) de ladite tige (107), ceux-ci étant pris par ladite tige (107) entre deux moments prédéfinis.

17. Appareil selon la revendication 16, **caractérisé en ce que** les produits de légumes (103) sont mis en mouvement par un premier moyen d'entraînement comprenant une chaîne mobile le long d'un chemin en boucle fermée agencé sur un plan vertical, et adaptée pour coulisser le long de ladite étendue de mesure (A), et **en ce que** ledit cylindre (105), ledit guide (102) ainsi que les éléments et les moyens qui leur sont associés sont entraînés de façon à se déplacer de façon synchrone avec lesdits berceaux respectifs, au moyen d'un second moyen d'entraînement qui est de préférence constitué par une seconde chaîne en boucle fermée se déplaçant de façon synchrone, et **en ce que** lesdits moyens de déplacement dans ladite étendue de mesure se placent eux-mêmes dans une position qui est sensiblement au-dessus de l'un respectif desdits berceaux (4).

18. Appareil selon l'une quelconque des revendications précédentes 14 à 17, **caractérisé en ce qu'**il est pourvu de moyens adaptés pour forcer sélectivement lesdits bouts de doigt à retourner dans une position rétractée, ainsi que des moyens supplémentaires adaptés pour empêcher la main mécanique d'être capable de réaliser tout mouvement de ré-ascension, après qu'elle a achevé sa descente vers le produit respectif à mesurer et qu'elle est venue se reposer par gravité sur ce dernier.

19. Appareil selon l'une quelconque des revendications précédentes 12 à 18, **caractérisé en ce que** ledit guide (102) supportant ladite main mécanique (101) est pourvu d'une rotule (15C) adaptée pour permettre la rotation et l'adaptation automatique de la position de ladite main mécanique à l'extrémité dudit guide.

20. Procédé de mesure de la valeur de croissance d'une portion de la surface d'un produit de légume ou fruit, **caractérisé en ce qu'**il comprend au moins une première phase dans laquelle un dispositif de mesure s'approche du produit à mesurer, une seconde phase dans laquelle le produit est soumis à une compression commandée de façon à forcer ledit dispositif de mesure à pénétrer en partie la surface du produit, et une troisième phase dans laquelle ladite pénétration est mesurée, dans lequel ;
- dans ladite première phase, ledit dispositif de mesure devient synchronisé sur un produit respectif, en restant séparé de ce dernier et agencé verticalement au-dessus de ce dernier jusqu'à ce qu'une position prédéfinie soit finalement atteinte ;
- dans ladite seconde phase, une partie dudit dispositif de mesure est pressée avec une force commandée contre au moins une portion de la surface du produit de façon à être capable de le pénétrer partiellement, en déplaçant de façon synchronisée ledit dispositif de mesure et le produit respectif d'une manière uniforme et continue, parallèle le long d'une même étendue « A » et par conséquent selon un temps / une longueur prédéfini(e),
- dans ladite troisième phase, la valeur selon laquelle ledit dispositif de mesure a pénétré le produit est mesurée automatiquement et le résultat d'une telle mesure est traité de façon à définir une caractérisation du produit respectif et à l'évaluer en conséquence.
